# EUROPEAN PATENT APPLICATION

(11) **EP 3 848 451 A1**
(43) Date of publication of application: **14.07.2021**
(21) Application number: 19861036.2
(22) Date of filing: 11.09.2019
(51) Int. Cl.: C12M 3/06, C12M 1/12, C12N 1/02, C12M 3/00

(54) **SEPARATION DEVICE AND METHOD FOR SEPARATING TO-BE-SEPARATED MATERIAL USING SAME**

(30) Priority: 11.09.2018 JP 2018170092
(71) Applicant: Nissan Chemical Corporation, Tokyo 103-6119 (JP)
(72) Inventor: HATANAKA, Daisuke, Funabashi-shi, Chiba 274-0052 (JP); KIDA, Katsuhiko, Shiraoka-shi, Saitama 349-0294 (JP); KANAKI, Tatsuro, Shiraoka-shi, Saitama 349-0294 (JP); HAYASHI, Hisato, Tokyo 103-6119 (JP)
(74) Representative: J A Kemp LLP
(86) International application number: PCT/JP2019/035651
(87) International publication number: WO 2020/054755

(57) **Abstract**

A separation device for separating a solid separation target contained in a suspension obtained by suspension culture of adherent cells by using a fine scaffold material, and a separation method. The separation device has a separation chamber having a first chamber and a second chamber, and the first chamber and the second chamber are divided by a mesh structure for separation. The mesh structure for separation has mesh-holes with a predetermined size to suppress passage of the separation target, and a mesh structure for separation is configured in the separation chamber such that the liquid in the aforementioned suspension has a vertically upward directional component in the advancing direction of the liquid when the liquid passes through the mesh-holes.

## Description

### [Technical Field]

The present invention relates to a separation device for separating, from a liquid, a separation target in a suspension obtained by suspension culture of adherent cells which is performed using a fine scaffold material, and use thereof.

### [Background Art]

In recent years, a technique of culturing animal and plant cells and/or tissues in a floating state has been reported in which nanofibers composed of polysaccharides (water-insoluble polysaccharides such as chitin and the like) with improved dispersibility in water are dispersed in a liquid medium and used as a fine scaffolding material (patent document 1) .

By using the aforementioned nanofiber as a scaffold material, and culturing adherent cells such as mesenchymal stem cells and pre-adipocytes while being adhered to the nanofibers, the cells can be suspension-cultured in a stationary state. The nanofiber is insoluble in a liquid medium, floats in the liquid medium, and has the activity of adhering cells. In suspension culture using the nanofiber, therefore, mesenchymal stem cells and preadipocytes adhered to the nanofiber exhibit long-term viability and proliferate well on the nanofiber while maintaining the phenotype thereof.

### [Document List]

### [Patent document]

patent document 1: WO 2015/111686

### [Summary of Invention]

### [Technical Problem]

The aforementioned nanofiber is biodegradable and can be degraded by an enzyme (polysaccharide-degrading enzyme) that degrades the polysaccharide (e.g., chitin) that constitutes the nanofiber. The present inventors took note of this point and found that, by applying the aforementioned polysaccharide-degrading enzyme to the nanofiber to which the cultured cells are adhered in a liquid medium, the cultured cells can be isolated without using a protease, that is, while suppressing damage to the cultured cells.

However, when the aforementioned cultured cells that have grown while being adhered to the nanofiber are isolated using a polysaccharide-degrading enzyme, a process of sufficiently decomposing the aforementioned nanofiber with the polysaccharide-degrading enzyme and precipitating and collecting the remaining cultured cells is required. It was found that such recovery process is associated with further problems that a large amount of a polysaccharide-degrading enzyme is required for culturing a large amount of cells and that undegraded polysaccharides remain and are mixed with the recovered cultured cells. It was also found that a process that utilizes descending of cultured cells due to their own weight in a liquid medium is not preferable because it takes a long time for recovery, and a process that utilizes centrifugation is not preferable because the cultured cells are damaged.

On the other hand, in cell culture, humoral factors produced by cultured cells (substances secreted by cultured cells in a liquid medium, substances obtained by lysing cultured cells, and the like) may be the target to be recovered. For example, when a substance secreted in a liquid medium by the cells attached to a fine scaffold material floating in the liquid medium is recovered, it is necessary to separate (scaffold material and cultured cells attached thereto) and (liquid medium), and recover the liquid medium. When a substance contained in cultured cells is recovered, it is necessary to dissolve the cultured cells in a liquid medium with a reagent, separate (scaffold material) and (liquid medium in which the cultured cells are dissolved), and recover the (liquid medium in which the cultured cells are dissolved).

When a liquid is a passing target as mentioned above, a simple recovery method that does not use centrifugation may be a method including passing only the liquid through a porous filter and recovering the target. However, it was found that, in the method using a porous filter, since the filter is quickly clogged by (scaffold material and cultured cells attached thereto) or (scaffold material), the filter needs to be replaced frequently, and efficient recovery of the liquid medium alone is difficult.

The purpose of the present invention is to provide a separation device that solves the aforementioned problem, more efficiently separates a separation target in a suspension culture of adherent cells using a fine scaffold material, and can obtain the passing target, and a separation method of a separation target using the same.

### [Solution to Problem]

The main constitution of the present invention is as follows.
[1] A separation device for separating a solid separation target contained in a suspension which is obtained by suspension culture of adherent cells by using a fine scaffold material, the separation device comprising:
   a separation chamber which comprises at least a first chamber and a second chamber, wherein the first chamber and the second chamber are divided by a mesh structure for separation, and an internal flow path is configured such that the liquid in the suspension medium that has entered the separation device moves from the first chamber through the mesh structure for separation to the second chamber and thereafter exits the separation device,
   the mesh structure for separation has mesh-holes with a predetermined size to suppress passage of the separation target, and
   the mesh structure for separation is configured in the separation chamber such that the advancing direction of the liquid in the aforementioned suspension has a vertically upward directional component when the liquid passes through the mesh-holes.
[2] The separation device according to [1], wherein
   the suspension comprises a solid passing target to be passed through the mesh structure for separation together with the liquid, and
   the mesh structure for separation has mesh-holes of a predetermined size that suppress passage of the separation target and allow passage of the passing target.
[3] The separation device according to [2], wherein
   the liquid in the suspension is the liquid medium, the separation target is a scaffold material detached from the cells in the liquid medium by a reagent that separates the scaffold material and the adherent cell, and the passing target is the adherent cells detached from the scaffold material.
[4] The separation device according to [3], wherein
   the scaffold material is a nanofiber composed of a water-insoluble polysaccharide, and
   the adherent cell is a cell selected from the group consisting of a dog kidney renal tubule epithelial cell (MDCK cell), a Chinese hamster ovary cell (CHO cell), and a mesenchymal stem cell (MSC).
[5] The separation device according to [2], wherein
   the liquid in the suspension is the liquid medium, the separation target is a scaffold material to which adherent cells suspension-cultured in the liquid medium are attached, and the passing target is a humoral factor released from the cells into the liquid medium by suspension culture.
[6] The separation device according to [2], wherein
   the liquid in the suspension is a liquid containing a cell lysate of suspension-cultured adherent cells, the separation target is a scaffold material left in the liquid by lysis of the cells, and the passing target is a substance derived from the adherent cell and released into the liquid by the lysis of the adherent cell.
[7] The separation device according to [1] - [6], wherein the mesh structure for separation is sheet-like, or has a shape of a bag.
[8] The separation device according to [1] - [7], wherein the first chamber comprises
   a first inlet port for flowing the suspension into the first chamber from the outside,
   a second inlet port for flowing a washing solution or reagent into the first chamber from the outside, and
   an outlet port for flowing the separation target from the first chamber to the outside.
[9] The separation device according to [1] - [8], wherein the second chamber is located on the first chamber,
   the first chamber has at least an inlet port for flowing the suspension from the outside, and the inlet port is provided on a side wall of the first chamber.
[10] The separation device according to [9], wherein a central axis of the inlet port is at a height of not less than 40% of the whole height of the inside of the first chamber.
[11] The separation device according to [9] or [10], further comprising one or more chambers stacked upwards as separation chambers on the second chamber, wherein
   a chamber adjacent to the second chamber in the one or more chambers is divided from the second chamber by a further mesh structure for separation,
   when the one or more chambers are two or more chambers, chambers adjacent to each other above and below are divided by a further different mesh structure for separation, and
   mesh-holes of a mesh structure for separation located higher have a smaller opening area to suppress more the passage of the separation target.
[12] The separation device according to [11], wherein the suspension comprises a solid passing target to be passed through the mesh structure for separation together with the liquid, and
   a size of the mesh-holes of the mesh structure for separation located at the top is determined in advance to allow for the passage of the separation target.
[13] A method for separating a solid separation target from a suspension by using the separation device of any of [1] - [12], wherein the suspension is obtained by suspension culturing adherent cells by using a fine scaffold material.
[14] The method according to [13], wherein
   the suspension comprises a solid passing target to be passed through the mesh structure for separation of the separation device together with the liquid,
   the mesh structure for separation has mesh-holes of a predetermined size to prevent the separation target from passing through and allow the passing target to pass through, and
   a step of recovering the passing target that passes through the mesh structure for separation is included.
[15] The method according to [13] or [14], wherein a flow velocity of the liquid in the suspension passing through the mesh structure for separation of the separation device is 0.01 mm/sec - 25 mm/sec.

### [Advantageous Effects of Invention]

In the separation device of the present invention (hereinafter to be also referred to as the separation device), the mesh structure for separation is configured such that the advancing direction of a liquid in a suspension has a vertically upward directional component when the liquid passes through mesh-holes. As a result, since the surface on the entrance side of the mesh structure for separation is an overhanging surface or downward-facing surface, the separation target that cannot pass through the mesh-holes and is trapped by the mesh structure for separation is detached from the mesh structure for separation due to the gravity and descends. For example, since a fine scaffold material used for suspension culture and the scaffold material with cells attached thereto tend to descend slowly in the liquid medium, when these are trapped by the mesh structure for separation, they tend to be detached from the mesh structure for separation due to the gravity and descend in the liquid. Therefore, clogging of the mesh structure for separation is suppressed, efficient separation or removal of the separation target becomes possible, and a passing target can be obtained efficiently.

When the scaffold material is a nanofiber composed of water-insoluble polysaccharides and the cultured cell is a mesenchymal stem cell, the usefulness of the separation device is remarkable. The nanofiber slowly descends and can be easily detached from cultured cells even without complete digestion, since an enzyme that decomposes water-insoluble polysaccharides is used. As a result, the damage on the cultured cells can be made smaller. When the separation device of the present invention is applied to the thus-obtained suspension (suspension in which nanofibers and cultured cells are separated from each other and contained in a liquid), the nanofibers can be efficiently separated from the suspension while clogging is being suppressed, and efficient recovery of the cultured cells with less damage becomes possible.

The separation device of the present invention can efficiently separate a solid separation target in a suspension while suppressing clogging not only in the aforementioned recovery of cultured cells but also in the recovery of a humoral factor released from cells into a liquid medium in suspension culture or in the recovery of a substance derived from cells released into the aforementioned liquid by the lysis of the cell. Thus, the usefulness thereof becomes obvious.

### [Brief Description of Drawings]

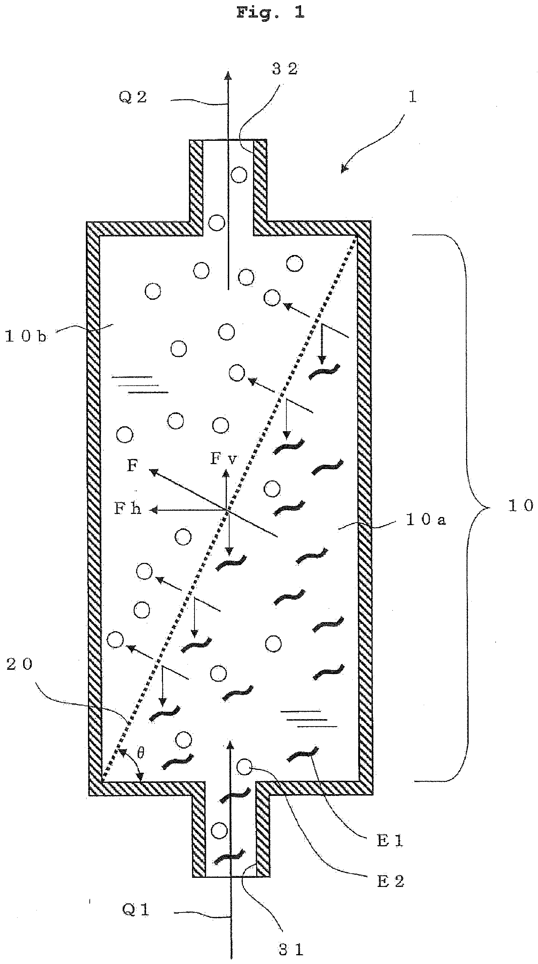

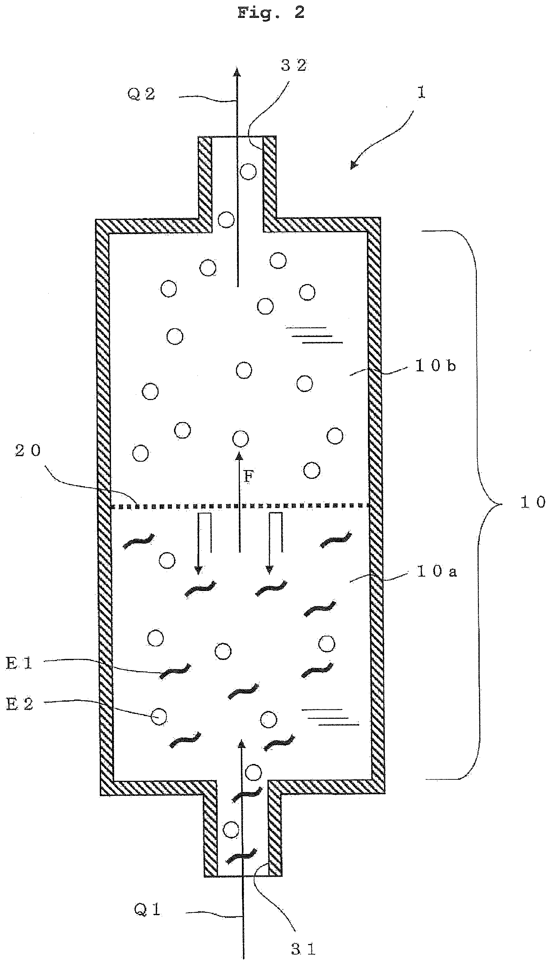

Fig. 1 is a sectional view schematically showing one embodiment of a preferred embodiment of the separation device of the present invention. In the Figure, the section of the mesh structure for separation is shown with a thick dotted line. The interval between the dots in the dotted line and the actual size of the mesh-holes are not related. The separation target is indicated by a wave-shaped symbol suggesting nanofiber as a scaffold material, and the passing target is indicated by a symbol with a white circular shape suggesting cultured cells detached from the scaffold material (same in Fig. 2, Fig. 9, Fig. 10).
Fig. 2 is a sectional view schematically showing another embodiment of a preferred embodiment of the separation device.
Fig. 3 is an end surface view schematically showing another embodiment of a preferred embodiment of the separation device. In this Figure, the wall of the separation chamber is drawn with a thick line to simplify the Figure. The separation target and the passing target are not shown.
Fig. 4 is a sectional view showing other embodiment of a preferred embodiment of the separation device.
Fig. 5 shows the structure of a mesh used as a mesh structure for separation. Fig. 5(a) is a partially enlarged view showing a predetermined region of the sheet surface of the mesh, and Fig. 5(b) is a cross-sectional view taken along the line X1-X1 of Fig. 5(a). In Fig. 5(b), the cross section of the wire is hatched.
Fig. 6 shows the constitution of a porous film used as a mesh structure for separation. Fig. 6(a) is a partially enlarged view showing a predetermined region of the sheet surface of the porous film, and Fig. 6(b) is a cross-sectional view taken along the line X2-X2 of Fig. 6(a). In Fig. 6(b), the cross section of the film part is hatched.
Fig. 7 shows another embodiment of a preferred embodiment of the separation device. Fig. 7(a) shows the inside of the separation device by removing the side wall, and Fig. 7(b) is a sectional view of Fig. 7(a) along X3-X3.
Fig. 8 shows another embodiment of a preferred embodiment of the separation device. Fig. 8(a) shows the inside of the separation device by removing the side wall. Fig. 8(b) is a perspective view schematically showing one embodiment of the constitution of the separation device of Fig. 8(a). Fig. 8(c) is a perspective view schematically showing other embodiment of the constitution of the separation device of Fig. 8(a).
Fig. 9 is a sectional view showing other embodiment of a preferred embodiment of the separation device.
Fig. 10 is a sectional view showing other embodiment of a preferred embodiment of the separation device.
Fig. 11 shows a block diagram and a piping diagram showing one embodiment of a cell culture system constituted using the separation device. In the Figure, the conduit and the direction of the flow of liquid are indicated by arrows.
Fig. 12 is a flowchart illustrating the process of recovering cells from a suspension by using the separation device.
Fig. 13 is a flowchart illustrating the process of recovering a liquid medium from a suspension by using the separation device.
Fig. 14 is a flowchart illustrating the process of recovering a liquid medium in which the cells are dissolved by using the separation device.
Fig. 15 is a photographic view showing the separation device produced in Example 1 of the present invention.
Fig. 16 is a microscopic photograph showing the results of Example 2 of the present invention.
Fig. 17 is a microscopic photograph showing the results of Example 3 of the present invention.
Fig. 18 is a microscopic photograph of the suspension obtained in each step of Step 1 to Step 4 in Example 5 of the present invention.

### [Description of Embodiments]

First, the separation device of the present invention is explained in detail.

The separation device of the present invention is used to separate a separation target in a solid contained in a suspension obtained by suspension culture of adherent cells by using a fine scaffold material. The separation target may be one to be discarded or utilized. In the embodiment shown in Fig. 1, for the sake of explanation, fine nanofiber to be the scaffold material in the suspension culture is the separation target, and the cell detached therefrom (suspension-cultured cells) is the passing target. As shown in Fig. 1, the separation device 1 has a separation chamber 10, and the separation chamber 10 has at least a first chamber 10a and a second chamber 10b. The first chamber 10a and the second chamber 10b are divided by a mesh structure for separation 20. In the embodiment shown in Fig. 1, the shape of the separation chamber when viewed from the lateral side is a circle with few inside-corners (dead space) where accumulation occurs; however, it may be an ellipse or a rectangle with an appropriate roundness, or the like. The first chamber 10a is provided with an inlet port 31 for the suspension Q1 to flow in, and the second chamber 10b is provided with an outlet port 32 for the liquid Q2 that has passed through the mesh structure for separation 20 to flow out. The entire inner flow path of the separation device is constituted such that the liquid enters the inside, passes from the first chamber 10a through the mesh-holes of the mesh structure for separation 20 to the second chamber 10b, and thereafter goes out to the outside. The mesh structure for separation 20 has mesh-holes of a predetermined size to suppress the passage of the separation target E1. The passing target E2 is described later. An important characteristic here is that a mesh structure for separation 20 is provided in the separation chamber 10 such that the advancing direction F of the liquid in the suspension when the liquid passes through mesh-holes of the mesh structure for separation 20 has a vertically upward direction component Fv. In the example of Fig. 1, the advancing direction F of the liquid medium also has a horizontal direction component Fh. In the example of Fig. 1, the mesh structure for separation 20 is a flat sheet-like mesh. The mesh structure for separation is diagonally disposed such that the surface on the second chamber 10b side of the mesh structure for separation is an inclined plane, and therefore, the surface on the first chamber 10a side is an overhang surface. The entire surface on the first chamber 10a side of the mesh structure for separation may not be an overhang surface, and the mesh structure for separation may be curved or bent to topically include a surface free from overhanging. The inlet port 31 does not necessarily have to be provided at the bottom of the separation chamber, and the outlet port 32 does not necessarily have to be provided at the top of the separation chamber. In the constitution of Fig. 1, when the inlet port 31 is provided in the upper part of the first chamber 10a and the outlet port 32 is provided in the lower part of the second chamber 10b, the advancing direction F of the liquid in the suspension, when the liquid passes through the mesh-hole of the mesh structure for separation 20, has a vertically-upward direction component Fv even when the inlet port 31 is provided at a position higher than the outlet port 32.

With the above constitution, the separation target E1 trapped on the surface on the first chamber 10a side of the mesh structure for separation 20 is detached from the mesh structure for separation and descends. As a result, clogging of the mesh-hole of the mesh structure for separation with the separation target E1 is suppressed, and the liquid and passage target E2 in the suspension can efficiently pass through the mesh structure for separation, and are taken out from the separation device and recovered.

In the present invention, the "suspension obtained by suspension culture of adherent cells by using a fine scaffold material" contains the following.
(A) a liquid in which adherent cells are dissolved or dispersed while being attached to a scaffold material (e.g., liquid in a suspension culture state, such liquid from which a predetermined amount of only the liquid medium is removed, and the like).
(B) a liquid in which adherent cells are detached from the scaffold material by a reagent that reduces the adhesiveness between the scaffold material and the adherent cells, and the cells and the scaffold material are independently dispersed
(C) a liquid in which adherent cells are dissolved by a cell dissolution reagent, whereby the scaffold material detached from the adherent cell and a substance derived from the dissolved adherent cell are dissolved or dispersed
(D) a liquid in which scaffold material is decomposed by a reagent that decomposes the scaffold material, whereby the cells detached from the scaffold material and the decomposed scaffold material are dispersed.

The separation device can be used to efficiently separate solids and allow only liquids to pass through, and the aforementioned suspension may contain a solid passing target to be passed through the mesh structure for separation together with the liquid. In the embodiment of Fig. 1, cell E2 is detached from the scaffold material E1 by a reagent as in the above-mentioned (B), and the cell E2 is the solid passing target. In such cases, the size of the mesh-holes of the mesh structure for separation 20 is determined in advance such that it not only suppresses the passage of the separation target E1 but also allows for the passage of the passing target E1.

As the suspension, the separation target, and the passing target, the following can be recited as examples.
(a) the liquid in the suspension is a liquid medium, and the separation target is a scaffold material with cells attached thereto. In this case, a solid passing target is not contained and, for example, it can be used for simple concentration of the separation target, or recovery of a humoral factor released from the cells into a liquid medium in suspension culture.
(b) the liquid in the suspension is the liquid medium, the separation target is a scaffold material detached from the cells in the liquid medium by a reagent that separates the scaffold material and the adherent cells, and the solid passing target is the cells detached from the scaffold material
(c) the liquid in the suspension is a liquid containing the cells dissolved by a reagent that dissolves cells, and the separation target is a scaffold material left in the liquid by the lysis of the cells. A solid passing target is not contained, but a substance derived from the cells released into the aforementioned liquid by cell lysis is the recovery target.

Examples of the adherent cells to be suspension-cultured, the liquid medium, the scaffold material for suspension culture, the reagent that separates the scaffold material and the adherent cells, the humoral factor to be released from the adherent cell into the liquid medium, the reagent that dissolves the cells, and the substance in the cells that is released into the liquid by cell lysis are shown below.

### (Suspension culture)

Adherent cells can be suspension-cultured (three-dimensional culture) by culturing the adherent cells being attached to a suitable scaffold material (described in detailed below) in a liquid medium. Suspending of cells in the present invention refers to a state where cells do not adhere to a culture container (non-adhesive), and whether the cell has descended is not questioned.

The temperature when cells are cultivated is generally 25 to 39°C (e.g., 37°C), preferably 33 to 39°C, for animal cells. The CO₂ concentration is generally 4 to 10% by volume in the culture atmosphere, and 4 to 6% volume is preferable. The culture period may be set as appropriately according to the object of the culture.

For suspension culture of adherent cells, culture vessels generally used for cell culture such as schale, flask, plastic bag, Teflon (registered trade mark) bag, dish, schale, dish for tissue culture, multidish, microplate, microwell plate, multiplate, multiwell plate, chamber slide, tube, tray, culture bag, roller bottle and the like can be used for cultivation. These culture containers are desirably low cell-adhesive so that the adherent cells attached to a scaffold material will not adhere to the culture container. As a low cell-adhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used.

When the medium needs to be exchanged, the cells are separated by centrifugation or filtration treatment, and a fresh medium can be added of the cells. Alternatively, the cells are appropriately concentrated by centrifugation or filtration treatment, and a fresh medium can be added to the concentrated liquid. For example, unlimitatively, the gravitational acceleration (G) of centrifugation is 100G to 400G, and the size of the pore of the filter used for the filtration treatment is 10 jam to 100 µm.

The adherent cells can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high density culture.

### (Adherent cell to be subjected to suspension culture)

"Adherent cell" is a cell that requires a scaffold such as a container wall and the like for survival and proliferation. The aforementioned adherent cell to be subjected to suspension culture is not particularly limited and, for example, stem cell, progenitor cell, somatic non-stem cell, primary cultured cell, cell line, cancer cell and the like can be mentioned. Stem cell is a cell concurrently having an ability to replicate itself, and an ability to differentiate into other plural lineages. Examples of the adherent stem cell include, but are not limited to, somatic stem cell and the like such as mesenchymal stem cell, neural stem cell, hematopoietic stem cell, liver stem cell, pancreas stem cell, muscle stem cell, reproductive stem cell, intestinal stem cell, cancer stem cell, hair follicle stem cell and the like. Mesenchymal stem cell is a stem cell having differentiation potency into all or some of osteocyte, chondrocyte and adipocyte. Mesenchymal stem cell is present in a tissue such as bone marrow, peripheral blood, cord blood, adipose tissue and the like at a low frequency and can be isolated from these tissues by a known method. Progenitor cell is a cell on the way to differentiate from the aforementioned stem cell into a particular somatic cell or reproductive cell. Examples of the adherent progenitor cell include, but are not limited to, pre-adipocyte, cardiac muscle progenitor cell, endothelial progenitor cell, neural progenitor cell, liver progenitor cell, pancreas progenitor cell, kidney progenitor cell and the like. Examples of the adherent somatic non-stem cell include, but are not limited to, fibroblast, osteocyte, bone pericyte, keratinocyte, adipocyte, mesenchymal cell, epithelial cell, epidermal cell, endothelial cell, vascular endothelial cell, hepatocyte, chondrocyte, cumulus cell, neural cell, glial cell, neuron, oligodendrocyte, microglia, astrocyte, heart cell, esophagus cell, muscle cell (e.g., smooth muscle cell or skeletal muscle cell), pancreas beta cell, melanin cell, and the like. Primary cultured cell is a cell after separation of cells and tissues from a living body and in a state of culture before performing the first passage. The primary cultured cell may be a cell collected from any tissue, for example, skin, kidney, spleen, adrenal gland, liver, lung, ovary, pancreas, uterus, stomach, colon, small intestine, large intestine, bladder, prostate, testis, thymus, muscle, bond tissue, bone, joints, blood vessel tissue, blood, heart, eye, brain, nerve tissue and the like. Cell lines are cells that have acquired infinite proliferative capacity by an artificial operation in vitro. Examples of the adherent cell to be used in the present invention include, but are not limited to, dog kidney renal tubule epithelial cell (MDCK cell), Chinese hamster ovary cell (CHO cell), mesenchymal stem cell (MSC), more preferably mesenchymal stem cell.

The origin of the adherent cell is not particularly limited, and the cell may be derived from either an animal or a plant. Examples of the animal include insect, fish, amphibian, reptiles, birds, pancrustacea, hexapoda, mammals and the like, with preference given to mammal. Examples of the mammal include, but are not limited to, rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like. The plant is not particularly limited as long as the collected cells can be applied to liquid culture. Examples thereof include, but are not limited to, plants (e.g., ginseng, periwinkle, henbane, coptis, belladonna etc.) producing crude drugs (e.g., saponin, alkaloids, berberine, scopolin, phytosterol etc.), plants (e.g., blueberry, safflower, madder, saffron etc.) producing dye or polysaccharide (e.g., anthocyanin, safflower dye, madder dye, saffron dye, flavones etc.) to be a starting material for cosmetic or food, or plants producing a pharmaceutical active pharmaceutical ingredient, and the like. In the present invention, mammalian adherent cells are preferably used.

### (Liquid medium)

The liquid medium that can be used for the aforementioned suspension culture can be appropriately selected according to the kind and the like of the adherent cells to be used. For example, when used for the purpose of culture of mammalian adherent cells, a medium generally used for culturing mammalian cells can be used as a medium to be contained in the medium composition of the present invention. Examples of the medium for mammalian cells include Dulbecco's Modified Eagle's Medium (DMEM), hamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Quality Biological), StemPro hESC SFM (manufactured by Invitrogen), mTeSR1 or 2 medium (manufactured by STEMCELL Technologies), Sf-900II (manufactured by Invitrogen), Opti-Pro (manufactured by Invitrogen), and the like.

### (Scaffold material for suspension culture)

The scaffold material for the aforementioned suspension culture is not particularly limited as long as it achieves suspension culture of adherent cells. Nanofibers composed of water-insoluble polysaccharides and microcarrier beads and the like are preferably used. A method in which cells are engrafted on the carrier surface of microcarrier beads and the medium containing the beads is stirred and cultured in a floating state is generally also referred to as "microcarrier culture", "suspension culture" or the like. In the present specification, such culture method is also included in the "suspension culture".

In the present specification, the "nanofiber" refers to a fiber having an average fiber diameter (D) of 0.001 to 1.00 µm. The average fiber diameter of the nanofiber to be used in the present invention is preferably 0.005 to 0.50 µm, more preferably 0.01 to 0.05 µm, further preferably 0.01 to 0.02 µm. The aspect ratio (L/D) of the nanofiber that can be used in suspension culture is not particularly limited and is obtained from average fiber length/average fiber diameter, and is generally 2 - 500, preferably 5 - 300, more preferably 10 - 250. Fibers in which these are associated as a quadratic aggregate are also included.

When suspension culture of adherent cells is performed using a nanofiber, adherent cells prepared separately are added to the medium and uniformly mixed. In this case, the mixing method is not particularly limited and, for example, manual mixing using pipetting and the like, mixing using instrument such as stirrer, vortex mixer, microplate mixer, shaking machine and the like can be mentioned. After mixing, the obtained cell suspension may be cultured while being stood still, or cultured with rotation, shaking or stirring as necessary. The rotating speed and frequency can be appropriately set according to the object of those of ordinary skill in the art. For example, adherent cells are recovered from the passage culture, dispersed to a single cell or close thereto using an appropriate cell dissociation solution, the dispersed adherent cells are suspended in the medium, and this is subjected to suspension culture (preferably, suspension standing culture).

In a preferable embodiment, the nanofiber is, upon mixing with a liquid medium, dispersed in the liquid while maintaining the primary fiber diameter, substantially retains the cells attached to the nanofiber without substantially increasing the viscosity of the liquid, and shows an effect of preventing the cells from descending and attaching to a culture container. Without substantially increasing the viscosity of the liquid means that the viscosity of the liquid does not exceed 8 mPa·s. In this case, the viscosity of the liquid (that is, the viscosity of the following medium composition of the present invention) is not more than 8 mPa·s, preferably not more than 4 mPa·s, more preferably not more than 2 mPa·s. The viscosity of the liquid containing the nanofiber can be measured, for example, using a tuning fork vibration type viscometer (SV-1A, A&D Company Ltd.) under 25°C conditions.

Examples of the water-insoluble polysaccharides constituting nanofiber include, but are not limited to, celluloses such as cellulose, hemicellulose and the like; chitinous substances such as chitin, chitosan and the like, and the like. The water-insoluble polysaccharides are preferably cellulose, chitin or chitosan, more preferably chitin.

Cellulose is a natural polymer compound wherein D-glucopyranoses which are a 6-membered ring of glucose, are β-1, 4 glucoside bonded. As the starting material, for example, plant-derived cellulose such as lumber, bamboo, hemp, jute, kenaf, cotton, agricultural crops.food residue and the like, or cellulose of microorganism production or animal production such as bacterial cellulose, Cladophora, Glaucocystis, Valonia, Tunicate cellulose and the like can be used. In the plant-derived celluloses, very fine fibers called microfibrils are bundled to form higher structures in stages such as fibril, lamella and fibre cell. In addition, in bacterial cellulose, cellulose microfibrils secreted from bacterial cells and having the unchanged thickness form a fine net structure.

In the present invention, cellulose material having high purity such as cotton, bacterial cellulose and the like can be directly used. However, other plant-derived cellulose and the like are preferably used after isolation and purification. Cellulose preferably used in the present invention includes cotton cellulose, bacterial cellulose, kraft pulp cellulose, crystalline cellulose and the like.

The chitinous substance refers to one or more carbohydrates selected from the group consisting of chitin and chitosan. Major sugar units constituting chitin and chitosan are N-acetylglucosamine and glucosamine, respectively. Generally, chitin has a high N-acetylglucosamine content and is poorly soluble in acidic aqueous solution, and chitosan has a high glucosamine content and is soluble in acidic aqueous solution. For convenience, chitin contains not less than 50% of N-acetylglucosamine in the constituent sugar, and chitosan contains less than 50% of N-acetylglucosamine in the present specification. To achieve a high suspending action, a higher ratio of N-acetylglucosamine in the sugar unit constituting chitin is more preferable. The ratio of N-acetylglucosamine in the sugar unit constituting chitin is preferably not less than 80%, more preferably not less than 90%, further preferably not less than 98%, most preferably 100%.

As the starting material of chitin, many biological resources such as shrimps, crabs, insect, shells, mushrooms and the like can be used. The chitin to be used in the present invention may be one having α-form crystal structure such as chitin derived from crab shell, shrimp shell and the like, or one having β-form crystal structure such as chitin derived from cuttlebones and the like. The test of crabs and shrimps is often regarded as industrial waste and preferable as a starting material since it is easily available and effectively used. On the other hand, it requires a protein removing step and a decalcification step to remove protein, minerals and the like contained as impurities. In the present invention, therefore, purified chitin that underwent a matrix removal treatment is preferably used. Purified chitin is commercially available.

By pulverizing the starting material containing the aforementioned water-insoluble polysaccharides, a nanofiber constituted of the water-insoluble polysaccharides can be obtained. While the pulverization method is not limited, a method affording a strong shear force such as a medium stirring mill, for example, a high-pressure homogenizer, a grinder (stone mill), a bead mill and the like is preferable for subdivision to the below-mentioned fiber diameter and fiber length meeting the object of the present invention.

Of these, subdivision by a high-pressure homogenizer is preferable, and, for example, subdivision (pulverization) by the wet grinding method disclosed in JP-A-2005-270891 or JP-B-5232976 is desirable. Specifically, the starting material is pulverized by spraying a dispersion of a starting material from a pair of nozzles at a high-pressure and bombarding each other, and, for example, Star Burst system (high-pressure pulverization device manufactured by Sugino Machine Limited) or NanoVater (high-pressure pulverization device of yoshida kikai co., ltd.) is used therefor.

In the subdivision (pulverization) of a starting material by the aforementioned high-pressure homogenizer, the degree of subdivision and homogenization depends on the pressure in pumping into an ultrahigh-pressure chamber in a high-pressure homogenizer, and the number (treatment number) of pass through the ultrahigh-pressure chamber, and the concentration of the starting material in the water dispersion. The pumping pressure (treatment pressure) is not particularly limited and it is generally 50 - 250 MPa, preferably 150 - 245 MPa.

While the concentration of the starting material in a water dispersion during the subdividing treatment is not particularly limited, it is generally 0.1 mass % - 30 mass %, preferably 1 mass % - 10 mass %. While the treatment number of the subdivision (pulverization) is not particularly limited, it varies depending on the concentration of the starting material in the aforementioned water dispersion. When the concentration of the starting material is 0.1 - 1 mass %, the treatment number of 10 - 100 is sufficient for pulverization, but 1 - 10 mass % sometimes requires about 10 - 1000 times of treatment.

The viscosity of the water dispersion during the aforementioned subdivision treatment is not particularly limited. For example, when the water-insoluble polysaccharide is α chitin, the viscosity of the water dispersion is within the range of 1 - 100 mPa·S, preferably 1 - 85 mPa·S (by tuning fork vibration type viscometer (SV-1A, A&D Company Ltd.) under 25°C conditions). The particle size of the water-insoluble polysaccharides in water dispersion during a subdivision treatment is not particularly limited. For example, when the water-insoluble polysaccharide is α chitin, the average particle size of α chitin in the water dispersion is within the range of 0.5 - 200 µm, preferably 30 - 150 µm (by laser diffraction/scattering type particle size distribution measurement apparatus LA-960 (Horiba, Ltd.)).

The preparation method of a nanofiber that can be used in suspension culture is described in WO 2015/111686 A1 and the likes.

The microcarrier beads that can be used for suspension culture are not particularly limited as long as they can engraft, maintain and proliferate adherent cells on the surface thereof, and known microbeads can be used. The shape of the beads may be spherical, prolate spheroid, ellipse or the like, but is not particularly limited. Examples of the microcarrier beads include, but are not limited to, glass beads, polystyrene beads, ceramic beads and the like. Microcarrier beads are commercially available, and the most suitable one may be appropriately selected according to the kind of adherent cells to be cultured, and the like.

When suspension culture of adherent cells is performed using microcarrier beads, adherent cells prepared separately are added to the medium containing the microcarrier beads and uniformly mixed. In this case, the mixing method is not particularly limited and, for example, manual mixing using pipetting and the like, mixing using instrument such as stirrer, vortex mixer, microplate mixer, shaking machine and the like can be mentioned. After mixing, the obtained cell cells suspension is cultured with rotation, shaking or stirring, whereby the microcarrier beads engrafted by cells are maintained in a floating state. The rotating speed and frequency can be appropriately set according to the object of those of ordinary skill in the art.

### (Reagent that separates a scaffold material and adherent cells)

The reagent that separates a scaffold material and adherent cells is not particularly limited as long as it can separate the scaffold material and the adherent cells. Examples of the reagent that separates a scaffold material and adherent cells include, but are not limited to, "a reagent for decomposing a scaffold material", "a reagent for reducing adhesiveness of cells" and the like. The reagent for decomposing the scaffold material is not particularly limited as long as it can decompose the scaffold material and detach the scaffold material and the adherent cells. For example, when cellulose nanofiber is used as the scaffold material, cellulase (endoglucanase (EC 3.2.1.4) ("EG"), exoglucanase or cellobiohydrolase (EC 3.2.1.91) ("CBH") or β-glucosidase ([β]-D-glucosideglucohydrolase; EC 3.2.1.21) ("BG")) can be used as the degrading enzyme thereof. When a cellulose material contains a large amount of hemicellulose, xylanase or mannanase is preferably added besides cellulose as an enzyme for degrading hemicellulose. For example, when chitin or chitosan is used as the water-insoluble polysaccharide, chitinase, chitobiase, chitosanase, β-1,3-glucanase and the like may also be used as the degrading enzyme thereof. A plurality of reagents that degrade scaffold material may also be used in combination. For example, when chitin or chitosan is used as the water-insoluble polysaccharide, a mixture of 2, 3 or 4 enzymes selected from the group consisting of chitinase, chitobiase, chitosanase and β-1,3-glucanase may also be used as the degrading enzyme thereof. Yatalase (Takara Bio Inc.) is a mixture of chitinase, chitobiase, chitosanase and β-1,3-glucanase, and can be preferably used as a degrading enzyme of chitin or chitosan.

For example, when the scaffold material is a nanofiber composed of water-insoluble polysaccharides, a degrading enzyme of the water-insoluble polysaccharides is added to a suspension of adherent cells attached to nanofibers composed of water-insoluble polysaccharides and the mixture is incubated for a time sufficient for the detachment of the adherent cells. For detaching, it is not always necessary to use a chelating agent (EDTA) or a protease (trypsin, collagenase) that can damage the cultured cells. The temperature of incubation by the degrading enzyme of the water-insoluble polysaccharides is generally 20°C - 37°C. The incubation time varies depending on the kind of the enzyme and the like and is generally 5 - 60 min. When the nanofiber is degraded and the adherent cells are detached from the nanofiber, the detached adherent cells can be recovered efficiently by subjecting the suspension to the separation device of the present invention.

Furthermore, the reagent that reduces the adhesiveness of the cell is not particularly limited as long as it reduces the adhesiveness of the cell. As such reagent, proteases such as trypsin, collagenase, hyaluronidase, elastase, pronase and the like, EDTA and the like can be used as a reagent that reduces the adhesiveness of the cell. For example, when the scaffold material is a microcarrier bead, the reagent is added to the suspension of adherent cells attached to the microcarrier beads and the mixture is incubated for a time sufficient to reduce adhesion of the adherent cells. The temperature of incubation of the suspension in the presence of the reagent is generally 20°C - 37°C. The incubation time varies depending on the kind of the enzyme and the like and is generally 5 - 60 min. When the adherent cells are detached from the scaffold material due to the reduction of the adhesiveness of the adherent cells, the detached adherent cells can be efficiently recovered by applying the separation device of the present invention.

### (Humoral factor released into liquid medium from adherent cell)

The humoral factor can be obtained by subjecting the adherent cells that produce a desired humoral factor to suspension culture while the cells are attached to a nanofiber composed of water-insoluble polysaccharides and separating the intended humoral factor from the culture (e.g., culture supernatant) by using the separation device of the present invention. Examples of the humoral factor include, but are not limited to, antibody, enzyme (urokinase etc.), hormone (insulin etc.), cytokine (interferon, interleukin, tumor necrosis factor, colony stimulating factor, growth factor etc.), vaccine antigen, and other physiologically active substances (protein, peptide etc.). The adherent cells that produce humoral factor includes untransformed cells such as skin cell, chondrocyte, hepatocyte, pancreatic cell, kidney cell, mesenchymal stem cell, adipocyte and the like, and transformed cells into which a gene encoding a humoral factor, or a gene involved in the biosynthesis of useful substances has been introduced. The adherent cell that produces desired humoral factor preferably secretes a humoral factor extracellularly. Specific examples of the adherent cell that produces humoral factor include, but are not limited to, HEK293, CHO-K1, BHK-21, MDCK, Vero, HepG2, MCF-7 and the like into which a gene encoding a humoral factor or a gene involved in the biosynthesis of a humoral factor has been introduced. The cells used for the production of a humoral factor such as recombinant protein and the like are well known to those of ordinary skill in the art.

### (Cell lysis reagent)

The cell lysis reagent is not particularly limited as long as it has an action of destroying or lysing the cell membrane and eluting a desired substance contained in the cell to the outside of the cell. It can be appropriately selected and used from compounds and compositions known in the field. Specific examples of the cell lysis reagent include, but are not limited to, organic solvent, chaotropic salt, surfactant and the like. Only one kind of reagent may be used as a cell lysis reagent, or two or more kinds of reagents may also be used.

### (Substance in cells released into liquid by lysis of adherent cell)

The substance released into the liquid by lysis of the cell includes all substances contained in the cell and is not particularly limited. Examples include proteins, viruses, nucleic acid molecules, and the like that are not secreted extracellularly from cultured adherent cells.

### (Placement attitude of mesh structure for separation)

In the example of Fig. 1, the mesh structure for separation is a mesh with a net-like part of sheet (i.e., thin plate), the surface (lower surface) on the first chamber 10a side of the mesh structure for separation is an overhang surface and is tilted by an elevation angle θ with respect to the horizontal plane. With the above constitution, the mesh structure for separation diagonally crosses the separation chamber, though depending on the shape of the separation chamber, the area (area of surface with mesh-holes) of the mesh structure for separation tends to become wider, which is preferable because the linear flow velocity can be reduced even when the flow rate is high. In addition, when the flow of the liquid that has entered the first chamber hits the mesh structure for separation at an angle, an action of easy delamination of the separation target attached to the mesh structure for separation by the flow of the liquid can also be expected.

On the other hand, in the example of Fig. 2, similar to the example of Fig. 1, the mesh structure for separation has a mesh with a net-like part of sheet. However, the surface (lower surface) on the first chamber 10a side of the mesh structure for separation is a horizontal plane (elevation angle θ of 0) faces directly downward (in other words, the advancing direction F of the liquid in the suspension when the liquid medium passes through the mesh-hole of the mesh structure for separation 20 has a vertically-upward direction component Fv alone). Such embodiment is preferable because all of the gravity acting on the separation target trapped in the mesh-hole acts on the separation target as a perpendicular detaching force to the lower surface of the mesh structure for separation, and the clogging can be cleared most effectively.

The angle between the lower surface of the mesh structure for separation and the horizontal plane (elevation angle θ in Fig. 1) may be 0 degrees or more and less than 90 degrees. As in the embodiment of Fig. 1, when the mesh structure for separation is diagonally arranged and the liquid is flown into the first chamber from below, a preferable range of the elevation angle θ is 45 degrees ≤ θ < 90 degrees because the effect that the separation target attached to the mesh structure for separation is easily detached by the flow of the liquid is expected and clogging with the separation target is suppressed. As described above, an embodiment in which the lower surface of the mesh structure for separation is a horizontal plane (elevation angle θ = 0 degrees) and an embodiment in which the lower surface of the mesh structure for separation is an oblique overhang surface have own advantages. Therefore, a preferred embodiment may be selected according to the use and the internal shape of the separation chamber.

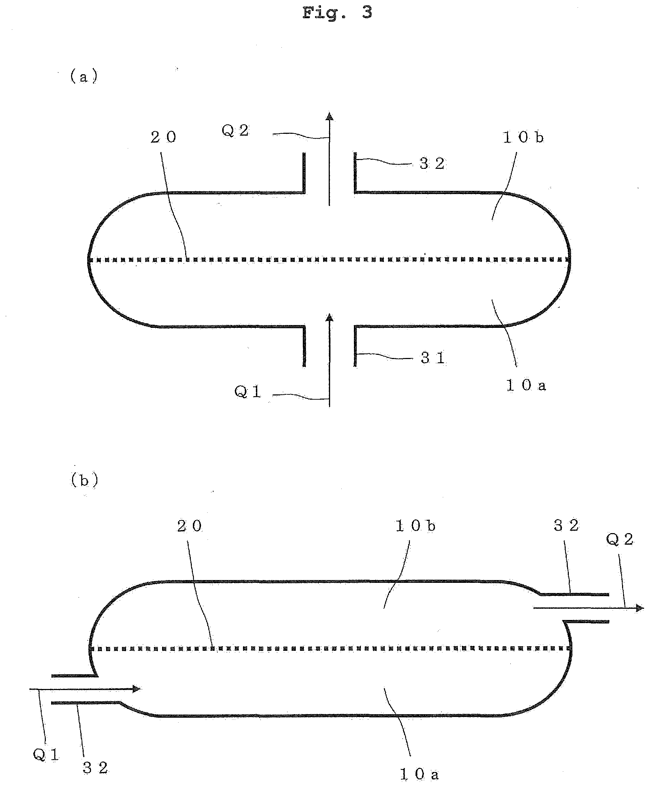

Fig. 3 is a variation of the embodiment of Fig. 2, in which an undersurface of the mesh structure for separation 20 is a horizontal plane (elevation angle θ is 0 degree), and the separation chamber spreads so that the area (area of surface with mesh-holes) of the mesh structure for separation will be wider. In the embodiment of Fig. 3(a), an inlet port 31 is provided in the center of the first chamber, an outlet port 32 is provided in the center of the second chamber, and a liquid enters the first chamber from directly below, passes through the mesh structure for separation to directly above, and goes out directly above from the second chamber. On the other hand, in the embodiment of Fig. 3(b), an inlet port 31 is provided on the side surface of the first chamber, an outlet port 32 is provided on a side surface of the second chamber on the opposite side from the inlet port 31, a liquid enters the first chamber from the lateral side, passes through the mesh structure for separation to directly above, and goes out to a lateral side from the second chamber. In the embodiment of Fig. 3, the shape of the separation chamber is a columnar shape with rounded corners (the central axis of rotation is the vertical direction of the paper surface), and the shape of the separation chamber is preferably appropriately determined so that the flow spreads over the entire surface of the mesh structure for separation.

### (Shape of mesh structure for separation)

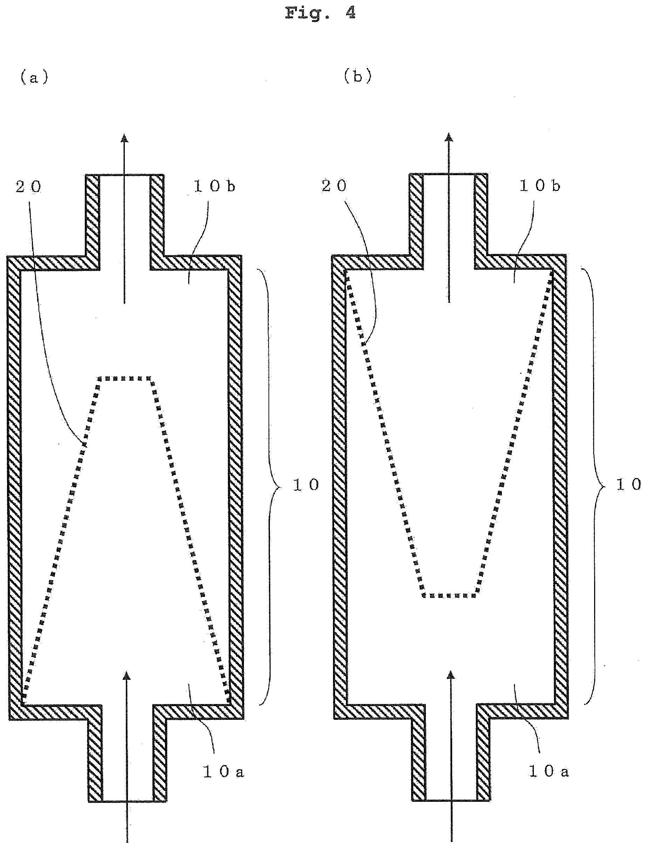

The shape of the mesh structure for separation (shape of mesh part) may be not only a flat sheet as shown in Fig. 1 and Fig. 2, but also a bag shown in Fig. 4.

In the embodiment shown in Fig. 4(a), the mesh structure for separation 20 is disposed such that the opening of the bag faces downward, the inside of the bag is a part or the entirety of the first chamber 10a, and the outside of the bag is the second chamber 10b. In the example of Fig. 4(a), the inside of the bag is the entirety of the first chamber 10a. When the position of the bag moves upward in the separation chamber, the inside of the bag becomes a part of the first chamber 10a. The inner surfaces of the wall of the bag, which face each other, are not parallel to each other, and the distance between the inner surfaces becomes narrower from the aforementioned opening toward the deeper inside of the bag. As a result, the inner surface of the bag is entirely an oblique overhang surface or a surface facing directly downward.

In the embodiment shown in Fig. 4(b), the mesh structure for separation 20 is disposed such that the opening of the bag faces upward, the outside of the bag is the first chamber 10a, and the inside of the bag is a part or the entirety of the second chamber 10b. In the example of Fig. 4(b), the inside of the bag is the entirety of the second chamber 10b. When the position of the bag moves downward in the separation chamber, the inside of the bag becomes a part of the second chamber 10b. The inner surfaces of the wall of the bag, which face each other, are not parallel to each other, and the distance between the inner surfaces becomes narrower from the aforementioned opening toward the deeper inside of the bag. As a result, the outer surface of the bag is entirely an oblique overhang surface or a surface facing directly downward.

### (Structure of mesh of mesh structure for separation)

The mesh structure for separation has a mesh-hole with a size predetermined to prevent a separation target from passing through (that is, being trapped). As a result, among the solids that have entered the separation device together with the liquid, a separation target of a size intended for separation cannot pass through the mesh-holes of the mesh structure for separation and accumulates in the first chamber. Therefore, the separation target accumulating in the first chamber can be separated. The separation target separated from the suspension may be one to be discarded or recovered for utilization.

The mesh structure for separation may be a mesh of knitted wire or a porous film with many through-holes formed on the film surface. For mesh, wires having various wire diameters and materials having various opening ratios are commercially available at low costs. In addition, a mesh made of a round wire (a wire having a circular cross-sectional shape) is preferable because a phenomenon that soft separation targets such as cell and the like are broken and pass through mesh-holes is suppressed and the possibility of receiving the targets becomes high. The mesh is described below, and the same constitution may be achieved using a porous film.

### (Equivalent-circle-diameter of opening shape of through-hole of mesh structure for separation)

The equivalent-circle-diameter of the opening shape of the through-hole (mesh-hole) of the mesh structure for separation varies depending on the separation target and passing target. For example, when the separation target is a granule or sphere used as the scaffold material in suspension culture, such as polystyrene bead and the like (e.g., CellBIND (registered trade mark) surface micro carrier (particle size 125 - 212 µm, manufactured by Corning), and the passing target is a cell detached from the scaffold material, the equivalent-circle-diameter of the opening shape of the through-hole is preferably about 20 µm - 200 µm, more preferably 20 µm - 100 µm, further preferably 20 µm - 50 µm, as a size that the bead cannot pass through. When the passing target does not exist and only a liquid is present, the equivalent-circle-diameter of the opening shape of the through-hole may be smaller.

When the separation target is a cell such as dog kidney renal tubule epithelial cell (MDCK cell), Chinese hamster ovary cell (CHO cell), mesenchymal stem cell (MSC) or the like, which is attached to the aforementioned bead, the passing target is absent, and only a liquid passes through, the equivalent-circle-diameter of the opening shape of the through-hole is, for example, about 1 µm - 20 µm.

When the separation target is a nanofiber composed of water-insoluble polysaccharide used as a scaffold material in suspension culture, such as MDCK cell, CHO cell, MSC and the like, and the passing target is a cell detached from the scaffold material, the equivalent-circle-diameter of the opening shape of the through-hole is preferably, for example, about 10 µm. - 200 µm, more preferably 20 µm - 100 µm, further preferably 20 µm - 50 µm. When the passing target is absent, and only a liquid is present, the equivalent-circle-diameter of the opening shape of the through-hole may be smaller.

When the separation target is a cell such as MDCK cell, CHO cell, MSC or the like, which is attached to the aforementioned nanofiber, the passing target is absent, and only a liquid passes through, the equivalent-circle-diameter of the opening shape of the through-hole is preferably, for example, about 1 µm - 200 µm, more preferably 5 µm - 100 µm, further preferably 10 µm - 50 µm.

The above sizes are examples in each separation target and passing target. They may be appropriately determined depending on the size of each separation target, the size of the passing target, and the presence or absence of the passing target so that the passage of the separation target will be suppressed.

While preferable sizes and the like of each part are exemplarily illustrated for cases where the adherent cell is MSC and the scaffold material is a nanofiber, other cells and scaffold material may also be changed to have appropriate sizes.

With only the definition of the aforementioned equivalent-circle-diameter, an elongated opening shape such as a slit and an intricate opening shape such as a maze are also included, and solids to be passed through other than the separation target are also entirely trapped.

Therefore, in the present invention, when only the scaffold material is separated, in addition to the aforementioned limitation on the equivalent-circle-diameter, the opening shape being a shape capable of accommodating a circle having a diameter of 10 µm to 200 µm (hereinafter referred to as contained circle) is added to the limiting condition. Here, the opening shape being able to accommodate the contained circle also includes the case where the contained circle is inscribed in the opening shape and the case where the contained circle is the same as the opening shape. The diameter of the contained circle is more preferably 20 µm - 100 µm, further preferably 20 µm. - 50 µm. When the opening shape is circular, the diameter of the contained circle = equivalent-circle-diameter, otherwise, the diameter of the contained circle <equivalent-circle-diameter.

Also, when a scaffold material with cells attached thereto is separated, in addition to the aforementioned limitation on the equivalent-circle-diameter, the opening shape being a shape capable of accommodating a contained circle having a diameter of 1 µm - 200 µm is also added to the limiting condition. The diameter of the contained circle is more preferably 5 µm. - 100 µm, further preferably 10 µm - 50 µm.

### (Opening shape of mesh-hole)

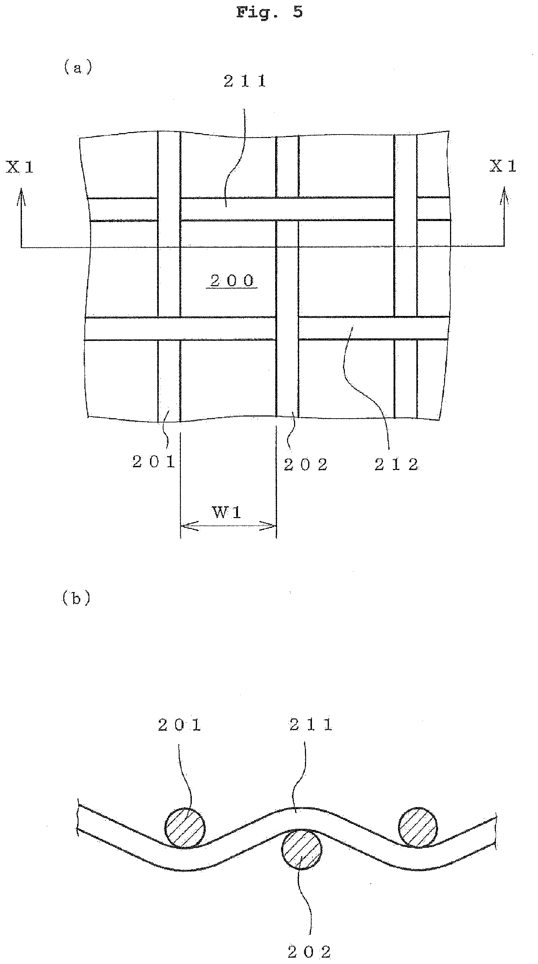

The opening shape of mesh-holes of the mesh structure for separation is not particularly limited, and may be triangular, quadrangular, hexagonal, or other polygonal or irregular shape, or the like. An opening shape of a general mesh which is economical and has high quality is, for example, a square. When the sheet surface of the mesh is seen in a straight view, the warp wire and the weft wire appear to intersect linearly as shown in Fig. 5(a), and the mesh-hole also looks like a flat plane square. When each mesh-hole is microscopically observed, since the warp wire and the weft wire are knitted three-dimensionally so as to avoid each other, four wires (two warp wires (201, 202) and two weft wires (211, 212)) constituting four sides surrounding one square mesh-hole 200 wave broadly in the thickness direction of the mesh as shown in Fig. 5(b). Of the four sides of the square, the distance W1 between two parallel sides facing each other may be the diameter of the aforementioned contained circle.

The diameter of the wire may be changed depending on the separation target, but it is generally the same and is preferably about 10 µm - 100 µm, more preferably 30 µm - 80 µm.

Examples of the material of the wire include nylon, polypropylene, polyethylene, polyester, PTFE, EVA, PPS, PEEK, Aramid, stainless steel and the like.

When the separation target is a cultured adherent cell (cell aggregate), the cell may be prevented from being divided by the wire by making the wire thicker, or the cell may be prevented from being divided by the wire by slowing the flow rate of the liquid passing through the mesh structure for separation. By increasing the volume of the separation chamber where the mesh structure for separation is arranged, making the cross-sectional area of the flow larger than the cross-sectional area of other conduits, or making the total opening area of the mesh-holes in the mesh structure for separation larger than the cross-sectional area of other conduits, the flow velocity of the liquid medium passing through the mesh structure for separation is decreased, and thus the division of cells by the mesh structure for separation is suppressed. As a result, the cells are trapped by the mesh structure for separation and accumulate in the first chamber 10a. The total opening area of the mesh-holes in the mesh structure for separation is preferably about 0.01-fold to 1000-fold, more preferably about 0.1-fold to 500-fold, the cross-sectional area of other conduits. For example, when the cross-sectional area of other piping tube is about 1 mm² - 50 mm², the total opening area of the mesh-holes in the mesh structure for separation is preferably about 0.01 mm² - 50000 mm².

### (Porous film)

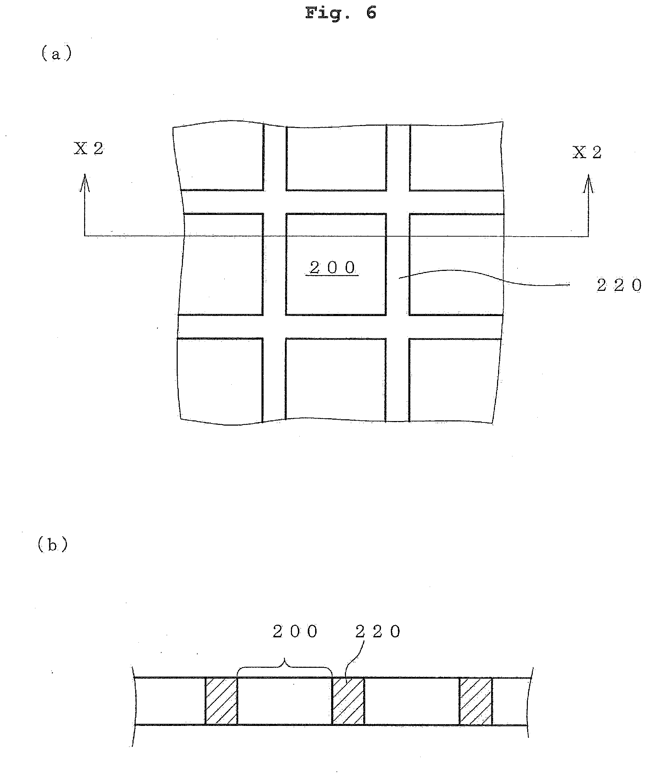

The mesh structure for separation may be a porous film. As shown in Fig. 6(a) and (b), the porous film is composed of a large number of through-holes 200 penetrating in the direction of the film thickness, and a beam part 220 which is a partition between the through-holes. Similar to the aforementioned mesh, the through-hole 200 has an opening shape of a size that suppresses passage of the separation target. The beam part 220 is a material part located between adjacent through-holes(rest of the film after subtracting through-holes). The beam parts are integrally connected so as to form a network. The opening shape, opening area, and beam part width of the through-hole 200 of porous film may be the same as the opening shape, opening area, and wire thickness of the above-mentioned mesh. Examples of the method for producing the porous film include resin molding, punching, etching on the film, electrocasting and the like.

### (Area of mesh structure for separation, volume of separation chamber)

The area of the mesh structure for separation (area of region facing the first chamber and where the mesh-holes exist) may be determined according to the use of the separation device (for small-scale experiments and large-scale industrial use), and is not particularly limited. It is preferably, for example, about 100 - 5000 mm² for small-scale experiments, and preferably about 5000 - 50000 mm² for large-scale industrial use.

The volume of the separation chamber may also be determined according to the use of the separation device and is not particularly limited. It is preferably, for example, about 1000 - 50000 mm³ for small-scale experiments, and preferably about 50000 - 500000 mm³ for large-scale industrial use.

### (Shape of separation chamber, position of port, mesh structure for separation)

The shape of the separation chamber is not particularly limited, and cylindrical shape, prismatic shape (cuboid), plate (circular plate, rectangle plate), bulk (spherical, ellipse spherical, shape close to cube), irregular shape and the like can be mentioned. The positions of the inflow port, mesh structure for separation, and the outlet port in such a separation chamber are not particularly limited. The internal flow path is preferably configured such that the liquid in the suspension that entered the separation device moves smoothly from the first chamber through the mesh structure for separation to the second chamber with lower resistance and then goes out of the separation device. In addition, the shape of the separation chamber and the position of the port are preferably determined such that the liquid in the suspension that entered the separation device spreads more than the mesh structure for separation and passes through the mesh structure for separation.

### (Material of wall part of separation chamber)

The material constituting the wall part of the separation chamber is not particularly limited, and examples thereof include organic polymer materials such as polystyrene, polypropylene, poly(ethylene terephthalate), polycarbonate, acrylic, silicon, polyvinylidene fluoride and the like, and metal materials such as stainless steel and the like. From the viewpoint of molding at a low cost and resistance to autoclaves and gamma rays, polypropylene, polycarbonate, and acrylic are exemplified as preferable materials.

### (Constitution for disposing mesh structure for separation in separation chamber)

The constitution for disposing the mesh structure for separation in the separation chamber is not particularly limited. For example, a constitution in which the first chamber and the second chamber are prepared as separate parts, and the mesh structure for separation is sandwiched and fixed by these parts, a constitution in which a mesh structure for separation with an adhesive margin is inserted into one separation chamber, the mesh structure for separation is adhered and fixed to the inner wall of the separation chamber using the adhesive margin, and the separation chamber is divided into a first chamber and a second chamber, and the like can be mentioned. Adhesion may be adhesion using an adhesive, welding of parts themselves, or the like.

### (Embodiment of the first chamber having plural inlet/outlet ports)

In the separation device, plural ports may be formed in each of the first chamber and the second chamber according to the purpose.

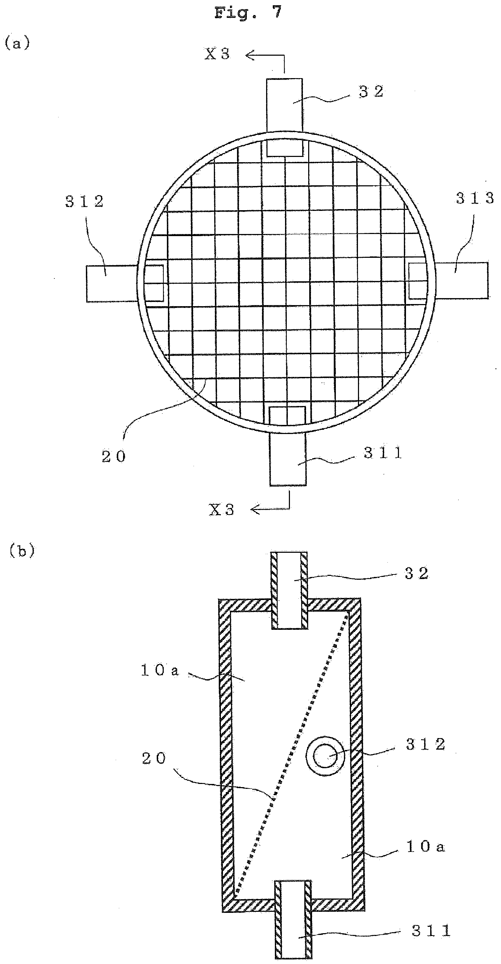

In the embodiment shown in Fig. 7, three ports (first inlet port 311, second inlet port 312, and outlet port 313) are provided in the first chamber, and an outlet port 32 alone is provided in the second chamber. In the embodiment shown in Fig. 7, the outer shape of the separation chamber is disk-like (cylindrical), and each port is located at a position with a space of an angle 90 degrees on the outer circumference side surface of the separation chamber.

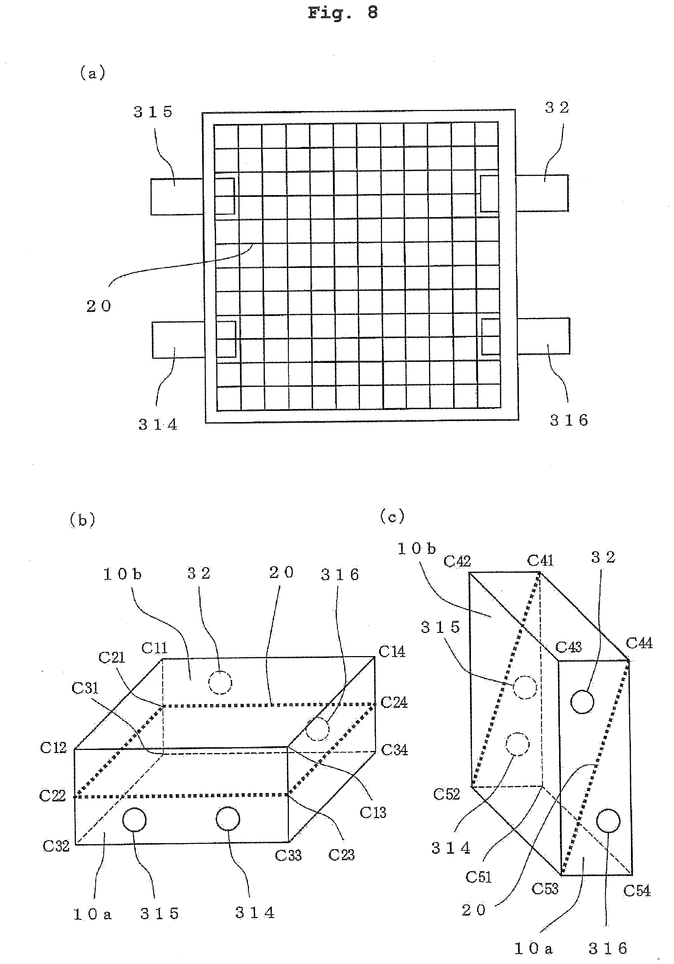

Similarly, in the embodiment shown in Fig. 8, three ports (first inlet port 314, second inlet port 315, and outlet port 316) are provided in the first chamber, and an outlet port 32 alone is provided in the second chamber. In the embodiment shown in Fig. 8, the shape of the separation chamber is cuboid-like (thin plate), and as for respective ports, two are provided on each of the two surfaces facing each other among the four surfaces on the outer circumference side surface of the separation chamber. More specific embodiment than the embodiment shown in Fig. 8(a) includes, for example, the embodiment shown in Fig. 8(b) and the embodiment shown in Fig. 8(c).

In the embodiment shown in Fig. 8(b), the separation chamber is cuboid-like (thin square plate laid horizontally) with eight vertices (C11, C12, C13, C14, C31, C32, C33, C34). The separation chamber is divided into a first chamber 10a on the lower side and a second chamber 10b on the upper side by a horizontally arranged quadrate sheet-like mesh structure for separation 20. The mesh structure for separation 20 has four vertices C21, C22, C23, C24. The first inlet port 314 and the second inlet port 315 in the first chamber are located in the side surfaces (C22, C23, C33, C32), and the outlet port 316 in the first chamber is located in the side surfaces (C21, C24, C34, C31). In addition, the outlet port 32 of the second chamber is located in the side surfaces (C11, C14, C24, C21).

In the embodiment shown in Fig. 8(c), the separation chamber is cuboid-like (thin square plate stood perpendicular) with eight vertices (C41, C42, C43, C44, C51, C52, C53, C54). The separation chamber is divided into a first chamber 10a on the lower side and a second chamber 10b on the upper side by a diagonally arranged quadrate sheet-like mesh structure for separation 20. The quadrate sheet-like mesh structure for separation 20 has four vertices C41, C52, C53, C44. The first inlet port 314 and the second inlet port 315 in the first chamber are located in the side surfaces (C41, C51, C52), and the outlet port 316 in the first chamber is located in the side surfaces (C44, C54, C53). In addition, the outlet port 32 of the second chamber is located in the side surfaces (C44, C53, C43) .

In the both embodiments shown in Fig. 7 and Fig. 8, the first inlet port is a port for allowing the suspension to flow into the first chamber from the outside. The second inlet port is a port for allowing a washing solution, a reagent and the like to flow into the first chamber from the outside. Examples of the aforementioned reagent include a cell detaching solution (e.g., a reagent capable of decomposing a scaffold material), a cell lysis reagent for lysing cells, and the like. The outlet port provided in the first chamber is a port for letting out the separation target left in the first chamber to the outside by the mesh structure for separation.

By providing these ports, an external reagent container can be connected to the first chamber of the separation chamber, and the scaffold material can be detached and cells can be lysed by applying the reagent in the first chamber. In addition, when another outside container is connected to the first chamber, the separation target remaining in the first chamber can be moved to the outside container, and it becomes possible to construct a flow path suitable for a closed culture system.

### (Position of inlet port in the first chamber)

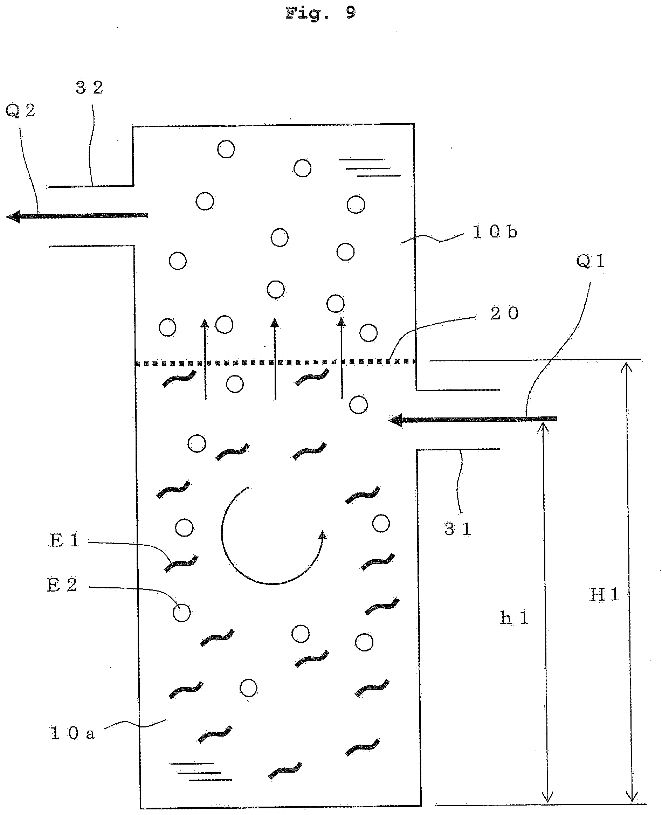

In the embodiment shown in Fig. 9, the second chamber 10b is located above the first chamber 10a, and the sheet-like mesh structure for separation 20 is horizontally arranged. Therefore, the advancing direction F of the liquid when passing through the mesh-holes of the mesh structure for separation 20 is a vertically upward direction.

In the embodiment shown in Fig. 9, the first chamber has at least an inlet a port 31 for the suspension Q1 to flow in from the outside, and the inlet port 31 is provided on the side wall of the first chamber. As a result, the bottom surface of the separation chamber becomes flat, thus permitting stable arrangement on the base surface.

In a preferred embodiment, the height h1 of the central axis of the inlet port is located at a height of 40% or more, preferably 60% or more, of the total height H1 in the room of the first chamber. As a result, a space exists in the first chamber below the height h1 of the central axis of the inlet port. Due to the presence of this space and the flow of the suspension Q1 flowing in from the inlet port provided on the side surface, the separation target and the passing target preferably circulate in the first chamber 10a, and the separation target is detached from the mesh structure for separation and can be accumulated in the space. The separation target accumulated in the space does not interfere with the passing target flowing in newly. Since the inlet port 31 is not provided on the bottom surface of the first chamber, the suspension flowing in newly does not blow up the separation target accumulated at the bottom of the space. Therefore, the passing target preferably passes through the mesh structure for separation. The upper limit of the height h1 of the central axis of the inlet port is a position where the upper end of the opening of the inlet port contacts the lower surface of the mesh structure for separation. That is, assuming that the diameter of the opening is d, the upper limit of the height h1 of the central axis of the inlet port is a position lowered by d/2 from the lower surface of the mesh structure for separation.

The position of the outlet port 32 provided in the second chamber is not particularly limited, and it may be provided on the side surface of the second chamber as in the embodiment shown in Fig. 9. In the embodiment shown in Fig. 9, the inlet port 31 provided in the first chamber and the outlet port 32 provided in the second chamber are provided on opposite sides of the side surface of the separation chamber, but they may be provided on the same side surface. The positions of the inlet port 31 and the outlet port 32 are preferably determined appropriately in consideration of the piping work and the piping space occupied by the tube.

### (Embodiment in which separation chamber is divided into three or more by two or more mesh structures for separation)

In the embodiment of Fig. 9, the separation chamber is divided into two by one mesh structure for separation. Above the second chamber, one or more chambers stacked upward may be further provided as separation chambers. A chamber adjacent to the second chamber in the aforementioned one or more chambers, and the second chamber are divided by a further mesh structure for separation. When the aforementioned one or more chambers are two or more chambers, the chambers adjacent to each other above and below are divided by a further different mesh structure for separation.

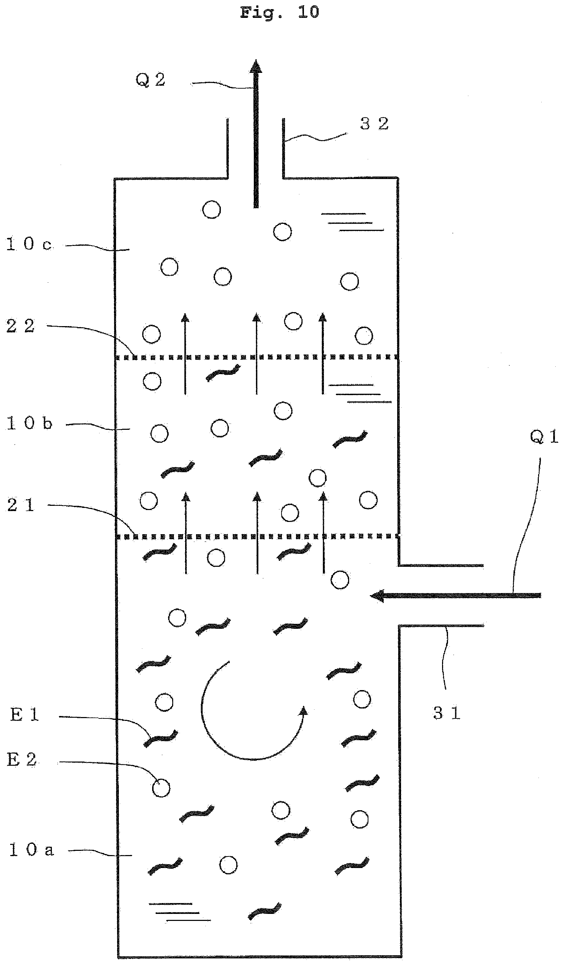

In the embodiment shown in Fig. 10, above the second chamber shown in Fig. 9, the chamber 10c stacked upward are further provided as separation chambers. The second chamber 10b is located above the first chamber 10a, a sheet-like mesh structure for separation 21 is horizontally provided between them, a third chamber 10c is located above the second chamber 10b, and a sheet-like mesh structure for separation 22 is horizontally provided between them. In other words, separation chambers are divided into three chambers 10a, 10b, 10c by two mesh structures for separation 21, 22. The outlet port is provided in the chamber (third chamber 10c) located at the top. In such constitution, mesh-holes of a mesh structure for separation located higher have a smaller opening area to suppress more the passage of the aforementioned separation target. As a result, the separation ability when the flow rate is increased (that is, the ability to stop the separation target without allowing passage thereof even when the flow rate is increased) is improved as compared with the case where the mesh structure for separation is single.

### (Embodiment 2 with plural structures layered up and down)

In the embodiment shown in Fig. 10, when separation target E1 alone is dispersed as a solid in suspension Q1, the lower limit of the size of the mesh-holes of the mesh structure for separation 22 located at the top is not particularly set. It is preferably a size that allows the liquid to be passed through to efficiently pass with less resistance.

On the other hand, in the embodiment shown in Fig. 10, when not only separation target E1 but also solid passing target E2 are contained as solids in suspension Q1, the size of the mesh-holes of the mesh structure for separation 22 located at the top is determined to be a size that allows passage of passing target E2 while suppressing the passage of separation target E1. Such embodiment in which the mesh structure for separation is provided in multiple stages is preferable since the separation performance does not decrease even when the flow rate is comparatively increased, because the mesh-holes of the first mesh structure for separation 21 is enlarged and coarse sieving can be performed to first remove only the large separation target during passage through the mesh structure for separation 21.

### (Cell culture system using the separation device of the present invention)

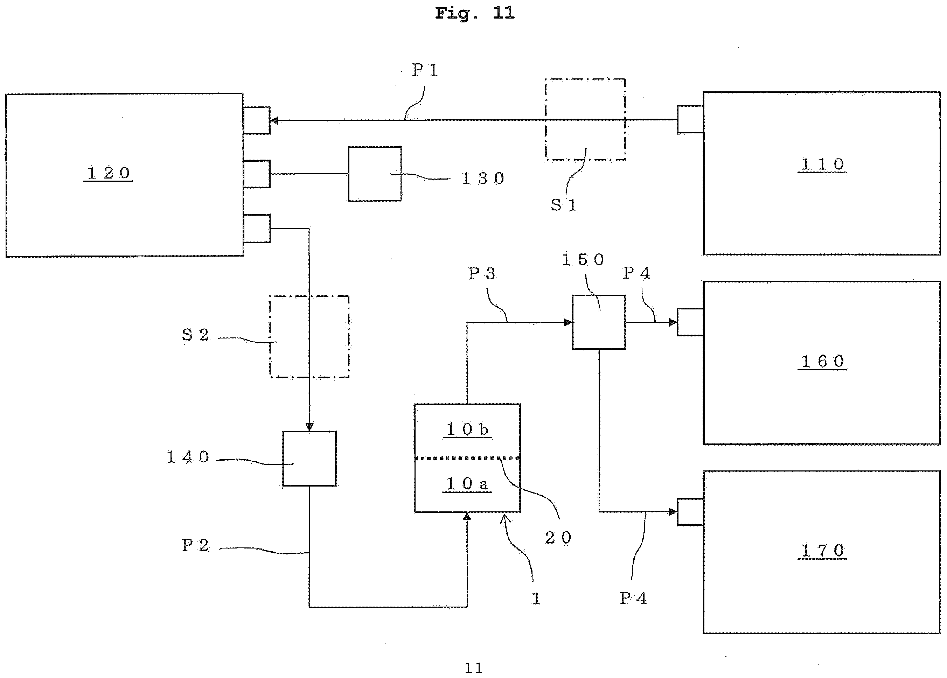

Fig. 11 shows a block diagram and a piping diagram showing one embodiment of a cell culture system constituted using the separation device. In the Figure, the conduit and the directions of the flow of liquid are indicated by arrows. In the constitution illustrated in Fig. 11, a scaffold material and the cells attached thereto are separated from the suspension by the separation device, only the liquid medium containing a humoral factor passes through and is recovered, or the scaffold material detached from the cells is separated from the suspension by the separation device, and only the liquid medium containing the cells passes through and is recovered. In the embodiment of the Figure, feed pumps S1, S2 are tube pumps, containers 110, 120, 160, 170 are flexible bags, a piping connecting the containers is a soft tube, and they enable cell culture, cell separation and recovery in a closed system where cells and liquid medium are not exposed to the outside air.

The liquid medium container 110 contains a liquid medium in which the scaffold material is dispersed. The liquid medium container 110 is connected to the cell culture container 120 via the piping P1, a feed pump S1 is provided on the pathway of the piping P1, a liquid medium in which the scaffold material (e.g., nanofiber containing chitin as a main component) is dispersed is sent to the cell culture container 120. On the other hand, a predetermined amount of adherent cells for seeding (e.g., MDCK cell, CHO cell, MSC) is injected into the cell culture container 120 from the cell injecting port 130, and suspension culture is performed in the cell culture container 120. The entire cell culture system is disposed in an incubator and kept at a suitable temperature (e.g., 33°C - 39°C) .

The cell culture system illustrated in Fig. 11 operates as follows.

First, when collecting only the liquid medium containing a humoral factor (e.g., INF-β, exosome), a suspension containing a scaffold material to which the cells are attached is sent to the first chamber 10a of the separation device 1 from the cell culture container 120 through piping P2. The scaffold material to which the cells are attached remains in the first chamber 10a, and only the liquid medium passes through the mesh structure for separation 20 to enter the second chamber 10b, and goes out of the separation device. The liquid medium is stored in a liquid (supernatant) recovery container 160 through piping P3, valve 150, piping P4. This procedure can also be used to concentrate cells (attached to the scaffold material) in the cell culture container 120. For example, in that case, the feed pump S2 is operated so that the feeding direction will be reversed, the concentrate remaining in the first chamber 10a is returned to the cell culture container 120, and the liquid medium is fed again from the liquid medium container 110, whereby the culture can be continued in the cell culture container 120.

Next, when the scaffold material is separated from the suspension-cultured adherent cells and the liquid medium containing the cells is recovered, a reagent (e.g., Yatalase) is fed into the cell culture container 120 from the reagent injecting port 140 to detach the scaffold material from the adherent cells. At this time, the feed pump S2 is operated so that the feed direction will be reversed. The suspension in which the scaffold material and the cells have been detached is sent from the cell culture container 120 to the first chamber 10a of the separation device 1 through the piping P2. The scaffold material is separated and remains in the first chamber 10a (the liquid medium also remains in the first chamber), and the cells and the liquid medium pass through the mesh structure for separation 20, enter the second chamber 10b, and go out of the separation device. The cells and the liquid medium are housed in cell recovery container 170 through piping P3, valve 150, piping P5.

These recovery operations may be performed by taking the cell culture system out of the incubator or in the incubator.

The system shown in Fig. 11 is merely one embodiment, and a required number of containers may be connected by piping such that the cultured cells pass through (or pass through the mesh structure for separation after entering the first chamber of the separation device) the mesh structure for separation of the separation device together with the liquid medium without directly contacting the outside air, and move between the containers (or return to the original container).

The container used in the above-mentioned cell culture system is not particularly limited, and a flexible bag used for cell culture and the like, a hard plastic or glass bottle or flask, and the like are exemplified as preferable ones. An appropriate stirring apparatus (e.g., shaker, paddle stirring and the like) may also be used depending on the culture scale.

The volume of each container can be determined according to the use of the system (for small-scale experiments or large-scale industries) and is not particularly limited. For example, about 10 mL (milliliter) - 1000 mL is exemplified as a preferable range for small-scale experiments, and about 1 L - 1000 L is exemplified as a preferable range for large-scale industries.

The feed pump is not particularly limited, and syringe, tube pump and the like are preferable.

When a gas permeation bag or a flask with a bent cap is used as a container, it is preferable to use an apparatus capable of controlling the internal temperature and gas concentration (particularly CO₂ concentration) as the incubator. When the container is a gas-impermeable closed container, an incubator that controls only the temperature can be used by controlling the CO₂ concentration in the liquid medium by exchanging the medium or the like.

### (Separation method)

Using the above-mentioned separation device of the present invention, a method for preferably separating a separation target in a solid contained in a suspension obtained by suspension culture of adherent cells by using a fine scaffold material can be provided. As described above, there are various combinations of the solid contained in the suspension, the target to be separated, and the target to be passed through. For example, when the liquid in the suspension is a liquid medium and the separation target is a scaffold material to which adherent cells are attached, a solid passing target is not contained and the method can be used for simple removal of the liquid (concentration) or recovery of a humoral factor released from the adherent cells into the liquid medium in suspension culture. The separation target may be a recovery target. When the liquid in the suspension is a liquid medium, and the separation target is a scaffold material detached from the cells in the liquid medium by a reagent that separates the scaffold material and the adherent cell, the passing target (recovery target) is adherent cells detached from the scaffold material. When the liquid in the suspension is a liquid containing the cells dissolved by a reagent that dissolves cells, and the separation target is a scaffold material left in the liquid by the lysis of the cells, the passing target (recovery target) is a substance derived from the cells released into the aforementioned liquid by cell lysis.

### (Flow velocity, flow rate)

The flow velocity when the liquid in the suspension passes through the mesh structure for separation of the separation device is not particularly limited. However, to improve separability and suppress clogging, it is preferably 0.01 mm/sec - 25 mm/sec, more preferably 0.1 mm/sec - 15 mm/sec, further preferably 1 mm/sec - 10 mm/sec.

The flow rate of the liquid passing through 1 cm² of the area of the mesh structure for separation (the area of the part that effectively acts as a net) is preferably 1 - 10 mL/min, more preferably 2 - 5 mL/min, further preferably 3 mL/min, to improve separability and suppress clogging. The below-mentioned Example 3 was performed at 30 mL/min, in which the area of the mesh structure for separation is about 12 cm², and the flow rate per 1 cm² area of the mesh structure for separation is 2.5 mL/min.

### (Separation and recovery Example 1: recovery of suspension-cultured MSC for transplantation)

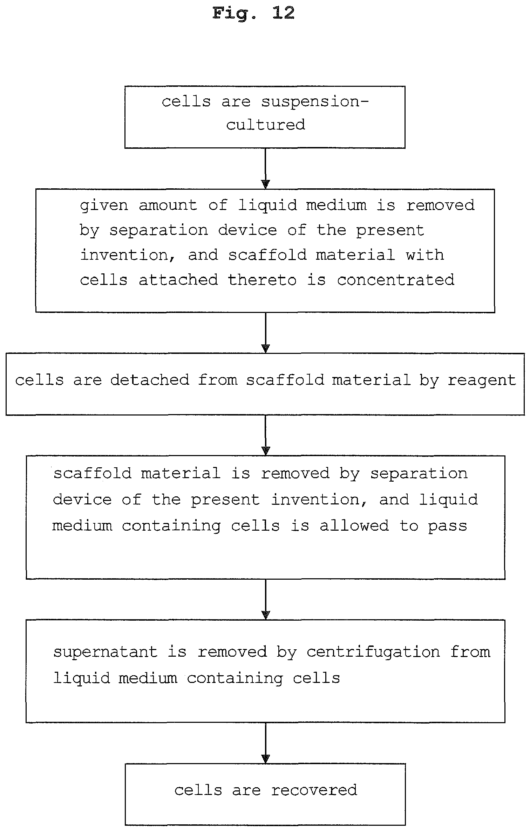

Fig. 12 is a flowchart showing one embodiment of the process when suspension-cultured MSC for transplantation is recovered using the separation device of the present invention.

As shown in the flowchart, cells are first suspension-cultured using a scaffold material (e.g., chitin nanofiber).

Then, the suspension (scaffold material to which the cells are attached is dispersed in the liquid medium) is fed into the separation device, whereby a given amount of the liquid medium is allowed to pass through and the scaffold material to which the cells are attached can be concentrated. The liquid medium (culture supernatant) that has passed through the separation device may be recovered.

Then, a solution containing an enzyme (e.g., Yatalase) that decomposes the scaffold material is injected, and the mixture is incubated for a given time to decompose the surface of the scaffold material, and the cells are detached from the scaffold material. It may be washed with PBS(-) (phosphate buffer) or the like before injecting the enzyme solution.

Next, the cells are collected by feeding a medium or PBS(-), trapping the scaffold material, and allowing only the cells to pass through. At this time, effective cell recovery is possible by feeding to the mesh surface of the mesh structure for separation in the direction opposite to the direction of gravity.

Then, the recovered solution is centrifuged and the supernatant is removed to recover only the cells.

### (Separation and recovery Example 2: recovery of liquid medium after suspension culture)

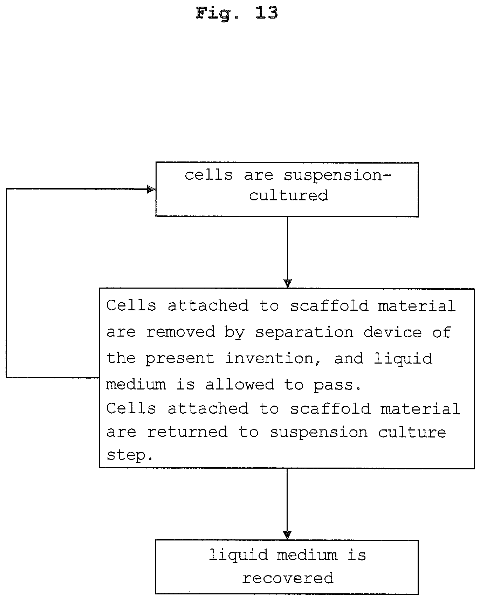

Fig. 13 is a flowchart showing one embodiment of the process of recovering a liquid medium after suspension culture by using the separation device of the present invention. As shown in the flowchart, adherent cells are first suspension-cultured using a scaffold material, and the suspension (scaffold material to which the adherent cells are attached is dispersed in the liquid medium liquid) is fed into the separation device of the present invention, whereby the scaffold material to which the cells are attached can be removed and the liquid medium (culture supernatant) can be recovered. The scaffold material to which the adherent cells are attached may be returned to the culture container and subjected again to suspension culture.

### (Separation and recovery Example 3: recovery of substance derived from cultured cells)

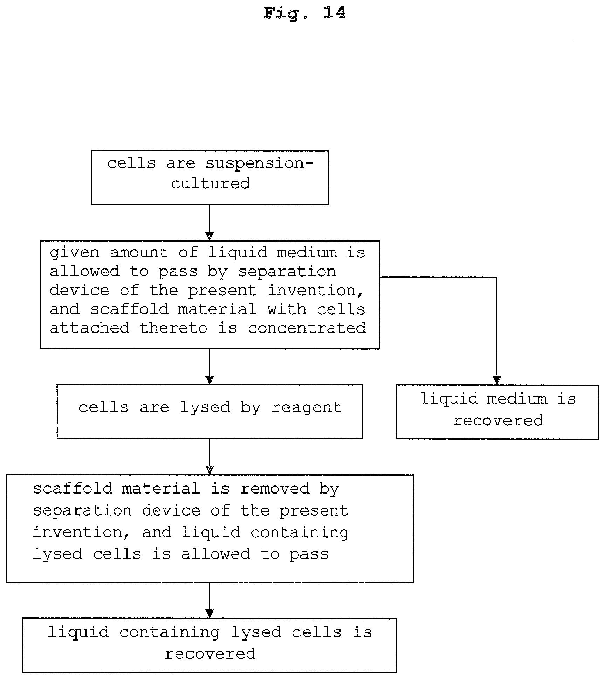

Fig. 14 is a flowchart showing one embodiment of the process of recovering a substance derived from cells cultured after suspension culture using the separation device of the present invention.

As shown in the flowchart, cells are first suspension-cultured using a scaffold material.

Then, the suspension (scaffold material to which the cells are attached is dispersed in the liquid medium) is fed into the separation device, whereby the liquid medium is allowed to pass through and the scaffold material to which the adherent cells are attached can be concentrated. The liquid medium (culture supernatant) that has passed through the separation device may be recovered as necessary.

Then, the cells are lysed by a cell lysis reagent. As a result, the suspension contains a scaffold material detached from the cells and cell lysate.

Then, the scaffold material is removed by the separation device of the present invention, a liquid containing a cell lysate is allowed to pass through, and the liquid is recovered.

### [Example]

### [Preparation Example 1]

### (Preparation of medium composition containing chitin nanofibers)

2 mass % chitin nanofiber (biomass nanofiber BiNFi-S, SUGINO MACHINE LIMITED CO., LTD.) prepared according to the description of the above-mentioned patent document 1 (WO 2015/111686) was mixed and suspended at 1%(w/v) in ultrapure water (Milli-Q water). Thereafter, the aqueous solution was autoclave sterilized at 121°C for 20 min. The obtained 1%(w/v) chitin nanofiber dispersion was added to and suspended in mesenchymal stem cellular proliferation medium 2 (#C-28009, manufactured by Takara Bio Inc.) (hereinafter sometimes to be indicate as the medium) to a final concentration of 0.01 or 0.02%(w/v), whereby a medium composition (hereinafter to be indicated as CNF medium composition) containing chitin nanofibers dispersed therein was prepared.

### [Example 1]

### (Manufacture of separation device)

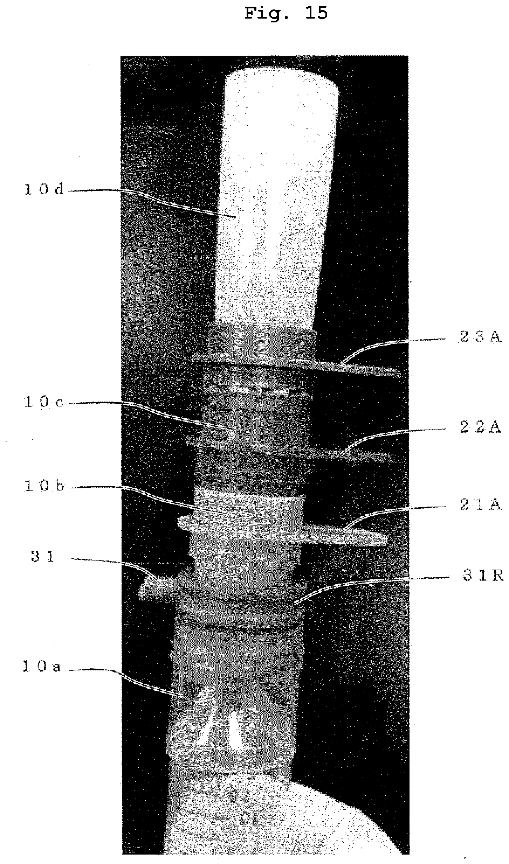

As shown in the photographic diagram of Fig. 15, a 50 mL conical tube (Falcon (R), manufactured by Corning Inc.) was cut into an upper part and a lower part at the position of the 45 mL scale, the lower part was turned upside down, and a conical-shaped part at the lower end was inserted into the upper side part from below, and the inside of the upper side part was used as the first chamber 10a.

A connector ring 31R having an inlet port 31 is connected on the aforementioned first chamber 10a, and three cell strainers (puriStrainer (registered trade mark) and manufactured by purriSelect Life Science) 21A, 22A, and 23A were mounted on the connector ring 31R. A truncated cone-shaped tube (Funnel, manufactured by purliSelect Life Science) 10d with openings at the top and bottom was further connected thereon to give the separation device of the present invention. The equivalent-circle-diameter of the quadrate openings of the mesh structure for separation (mesh) of the aforementioned three cell strainers are 100 µm, 60 µm, and 20 µm in order from the bottom.

In this separation device, the space between the mesh of the cell strainer 21A and the mesh of the cell strainer 22A is the second chamber 10b, and the space between the mesh of the cell strainer 22A, the mesh of the cell strainer 23A is the third chamber 10c, and the inside of the truncated cone-shaped tube 10d above is the fourth chamber. The separation device of this Example is similar to the separation device shown in Fig. 10 in the structural concept.

### [Example 2]

### (Separation of cells and scaffold material using separation device)

The separation of cells and scaffold material was examined using the separation device produced in Example 1.

Cultured human bone marrow-derived mesenchymal stem cells (C-12974, manufactured by Takara Bio Inc.) were suspended at 1000000 cells/10 mL in 0.02%(w/v) chitin nanofiber-containing medium composition produced in Preparation Example 1, and the suspension was filled in a 10 mL syringe (manufactured by TERUMO CORPORATION). This was connected to and injected into the port of the connector ring side part of the separation device.

Next, a 50 mL syringe (manufactured by TERUMO CORPORATION) filled with 40 mL of mesenchymal stem cellular proliferation medium 2 was connected to the port and the medium was injected at a flow rate of 0.5 mL/sec. As a result, the medium was flown from below while counteracting the direction of gravity with respect to the mesh.

The medium that was flown into the Funnel section was recovered, and the recovered solution was centrifuged (300×g, 3 min), and then the supernatant was removed. The medium (10 mL) was added to the remaining precipitate and it was resuspended, and the amount of ATP contained in the cells of the suspension was quantified by a plate reader (manufactured by Tecan) using CellTiter-Glo (Promega Corporation).

The cell recovery rate was calculated by comparing the RLU values obtained during the separation operation with the RLU value obtained by measuring the cell suspension after culturing and before the separation operation as the standard (cell recovery rate 100%).

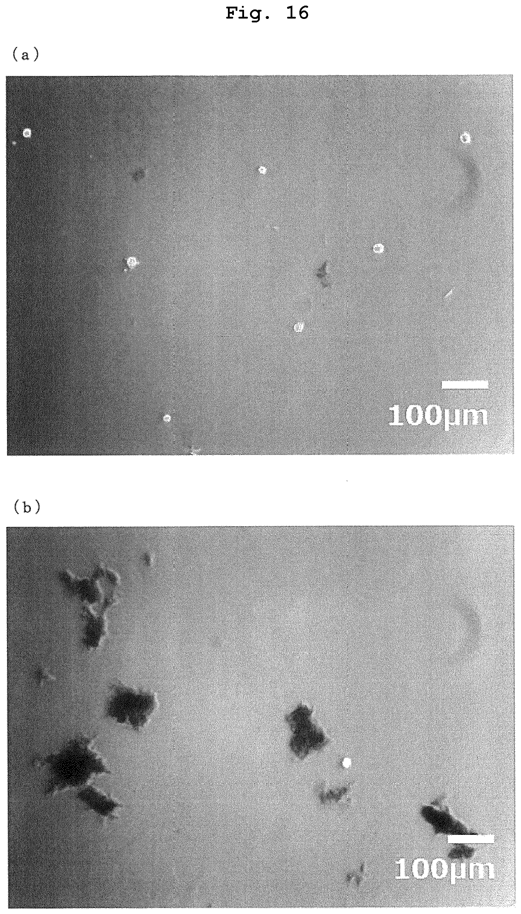

As a result, the cell recovery rate was 89%. Moreover, the suspension after recovery and the residue in the lower part of the separation device were observed with an inverted microscope. It was thus confirmed that almost no chitin nanofibers were observed in the suspension after recovery. Fig. 16(a), (b) show each microscopic photograph. Fig. 16(a) is a microscopic photograph of the solid in the liquid that passed through the separation device, and Fig. 16(b) is a microscopic photograph showing the solid (residue) in the liquid left in the first chamber of the separation device.

### [Comparative Example 1]

### (Separation of cells and scaffold material in cell strainer)

A connector ring was connected to a 50 mL conical tube, cell strainers of 20 µm, 60 µm, 100 µm and Funnel were mounted and fixed in order.

Cultured human bone marrow-derived mesenchymal stem cells (C-12974, manufactured by Takara Bio Inc.) were suspended at 1000000 cells/10 mL in 0.02%(w/v) chitin nanofiber-containing medium composition produced in Preparation Example 1, sucked by an electric pipette (Violamo Dispopipette), transmitted through the Funnel, and slowly discharged onto the cell strainer.

Then, an unfilled 10 mL syringe was connected to a port on the connector ring side part, and the piston of the syringe was slowly pulled to reduce the pressure inside the conical tube, and the liquid was passed through the cell strainer.

Then, 40 mL of mesenchymal stem cellular proliferation medium 2 was slowly poured onto the cell strainer through the Funnel, an unfilled 50 mL syringe was connected to the port on the connector ring side part, the piston of the syringe was slowly pulled to reduce the pressure inside the conical tube, and the liquid was passed through the cell strainer.

The medium in the conical tube was centrifuged (300×g, 3 min), and the supernatant was removed.

The medium (10 mL) was added to the remaining precipitate and it was resuspended, and the amount of ATP contained in the cells of the suspension was quantified by a plate reader (manufactured by Tecan) using CellTiter-Glo (Promega Corporation).

The cell recovery rate was calculated by comparing the RLU values obtained during the separation operation with the RLU value obtained by measuring the cell suspension after culturing and before the separation operation as the standard (cell recovery rate 100%). As a result, the cell recovery rate was 52%.

It is considered that this is because the chitin nanofibers covered the mesh and blocked the passage of the cells by filtering in order from the top. In addition, when the resuspended liquid was visually observed, floating materials different from the cells were observed. Thus, the contamination with chitin nanofibers was confirmed, and it was also found that the separation was insufficient. Therefrom the effectiveness of the separation recovery method performed in Example 2 using the separation device shape produced in Example 1 was shown.

### [Example 3]

### (Separation of cells and scaffold material using bag cell strainer)

Using a bone marrow fluid filtration filter (manufactured by KANEKA) which is one of the commercially available products, separation of cells and scaffold material was studied.

Cultured human bone marrow-derived mesenchymal stem cells (C-12974, manufactured by Takara Bio Inc.) were suspended at 1000000 cells/10 mL in 0.02%(w/v) chitin nanofiber-containing medium composition produced in Preparation Example 1, and the suspension was filled in a 10 mL syringe. This was connected to one end of the bone marrow fluid filtration filter and the liquid was sent from the lower part to the upper part direction.

Then, the syringe was replaced with a 50 mL syringe filled with 25 mL of the medium, the liquid was sent from the lower part to the upper part direction, and the medium coming out of the upper flow path was collected in a 50 mL conical tube.

The recovered solution was centrifuged (300xg, 3 min), and then the supernatant was removed.

The medium (10 mL) was added to the remaining sediment and resuspended, and the cell count was measured by a cell counter (TC-20, manufactured by Bio-Rad). As a result, the recovery rate was 86% as compared with the cell count before recovery.

In addition, the amount of ATP contained in the cells of the suspension was quantified by a plate reader (manufactured by Tecan) using CellTiter-Glo (Promega Corporation). The cell recovery rate was calculated by comparing the RLU values obtained during the separation operation with the RLU value obtained by measuring the cell suspension after culturing and before the separation operation as the standard (cell recovery rate 100%).

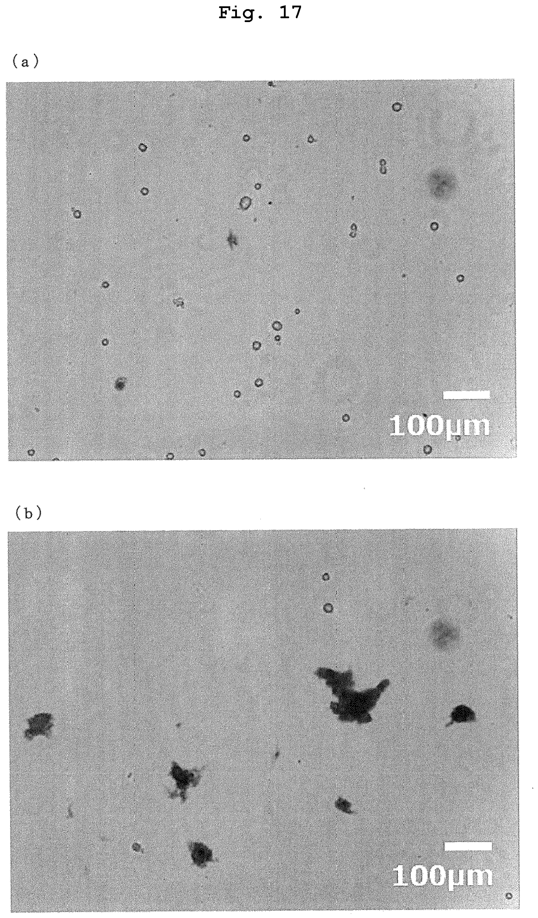

As a result, the cell recovery rate was 83%. Moreover, the suspension after recovery and the residue in the lower part of the separation device were observed with an inverted microscope. It was thus confirmed that almost no chitin nanofibers were observed in the suspension. Figs. 17(a), (b) show each microscopic photograph. Fig. 17(a) is a microscopic photograph of the solid in the liquid that passed through the separation device, and Fig. 17(b) is a microscopic photograph showing the solid (residue) in the liquid left in the first chamber of the separation device.

The results of the above-mentioned Examples 2, 4 and Comparative Example 1 are summarized in Table 1.

In Comparative Example 1, a large amount of scaffold material remaining in the recovered solution was observed, whereas in Examples 2 and 4, it was confirmed that there was almost no residue. In Examples 2, 4, the flow from the lower part made it difficult for the cells to come into contact with the outside air, drying of cells in the recovery process was prevented, and efficient recovery could be achieved.

**[Table 1]**

| | recovery rate (%) | remaining scaffold material *1 | air contact *2 |
|---|---|---|---|
| Example 2 | 89 | ○ | ○ |
| Example 3 | 83 | ○ | ○ |
| Comparative Example 1 | 52 | × | × |

| | | | |
|---|---|---|---|
| *1...○: Very small amount of scaffold material contaminated in the recovered solution, ×: large amount of scaffold material contaminated in the recovered solution *2...○: Low exposure to air, ×: constant exposure to air | | | |

### [Example 4]

Cultured human bone marrow-derived mesenchymal stem cells (C-12974, manufactured by Takara Bio Inc.) were seeded at 40000 cells/mL in the 0.01%(w/v) chitin nanofiber-containing medium composition produced in the above-mentioned Preparation Example 1, and non-addition medium, and dispensed into the wells of a 6 well flat bottom ultra low attachment surface microplate (manufactured by Corning Incorporated, #3471) at 2 mL per well.

Each plate was cultured in a CO₂ incubator (37°C, 5% CO₂) in a stationary state, and culture was continued for 5 days. On day 5, the cell recovery operation was performed. After culture, the cells/chitin nanofiber suspension were recovered in a 15 mL centrifuge tube, and centrifuged (220×g, 3 min).

The supernatant was removed, D-PBS (#045-29795, manufactured by FUJIFILM Wako Pure Chemical Corporation) (3 mL) was added, and the mixture was washed and centrifuged (220×g, 3 min) .

Successively, as a reagent that decomposes the scaffold material, Yatalase (#T017, manufactured by Takara Bio Inc.) was dissolved in D-PBS at 1%(w/v). A detach solution (sample 1) obtained by adding 1%(w/v) Yatalase solution (final concentration 0.4%) and Tryple select (1×) (#12563029, manufactured by Thermo Fisher Scientific) (final concentration 0.1×) to DMEM (low glucose) (#041-29775, manufactured by FUJIFILM Wako Pure Chemical Corporation), and a detach solution (sample 2) obtained by adding 1%(w/v) Yatalase solution (final concentration 0.4%) thereto were added with a 2 mL/15 mL centrifuge tube.

As a control sample, DMEM (low glucose) alone (2 mL) was added (sample 3). After suspending, the cells were seeded again in a 6 well flat bottom ultra low attachment surface microplate, and stood in a CO₂ incubator (37°C, 5%CO₂) for 1 hr.

Successively, the detached cells, chitin nanofiber and medium were recovered in a 15 mL centrifuge tube, and centrifuged (220×g, 3 min).

After removal of the supernatant, D-PBS (3 mL) was added, and the mixture was washed and centrifuged (220×g, 3 min). After removal of the supernatant, the medium (2 mL) was added, seeded in a 6 well adhesion surface microplate (manufactured by Corning Incorporated, #3516), and stood in a CO₂ incubator (37°C, 5%CO₂) for 3 hr. After 3 hr, the supernatant and chitin nanofiber were recovered and centrifuged (220×g, 3 min).

After removal of the supernatant, ATP reagent (500 µL) (CellTiter-GloTM Luminescent Cell Viability Assay, manufactured by Promega Corporation) was added.

The cells adhered to the 6 well adhesion surface microplate were washed by adding D-PBS (2 mL), removed and ATP reagent (500 µL) was added.

Using Enspire (manufactured by Perkinelmer), the luminescence value (RLU) was measured, and 100×(RLU on microplate)/(RLU+RLU of chitin nanofiber in microplate) was calculated, from which the recovery rate due to the detach solution was calculated. As a result, 78% and 69% of the cells could be respectively recovered in sample 1 and sample 2. On the other hand, the recovery rate was 5% in the control group free of the treatment with the detach solution.

From the above results, it was shown that the suspension-cultured adherent cells can be conveniently detached from a scaffold material by using a reagent that decomposes the scaffold material, and the detached cells can be efficiently recovered.

### [Example 5]

### (Series of studies from dispersion culture to separation of cells and scaffold material, to cell recovery)

### <<Step 1: culture step>>

Cultured human bone marrow-derived mesenchymal stem cells (C-12974, manufactured by Takara Bio Inc.) were seeded at 10000 cells/mL in the 0.01%(w/v) CNF-containing medium composition produced in the above-mentioned Preparation Example 1, and dispensed into 4 wells of a 6 well flat bottom ultra low attachment surface microplate (manufactured by Corning Incorporated, #3471) at 10 mL per well. Each plate was cultured in a CO₂ incubator (37°C, 5%CO₂) in a stationary state, and culture was continued for 5 days.

### <<Step 2: rough separation step of substrate and cells>>

The suspension (40 mL) after culture was recovered in a 50 mL conical tube, centrifuged (300×g, 3 min), and the supernatant (35 mL) was removed. An isotonic Percoll solution (10 mL) obtained by adding 10×PBS(-) (5 mL) to a density gradient adjusting solution (Percoll PLUS, manufactured by GE Healthcare) (45 mL) was added, mixed by pipetting, and centrifuged (400×g, 10 min), whereby the substrates free of cells precipitated and fraction of cells attached to the substrate remained on the upper layer. The upper layer (10 mL) was recovered in a 50 mL conical tube, diluted with PBS(-) (40 mL), centrifuged (400xg, 3 min), and washed by removing the supernatant (40 mL).

### <<Step 3: step of detaching cell from substrate>>

Then, an enzyme for detaching cells (Accumax, manufactured by Nacalai Tesque) (10 mL) was added, and the mixture was stood in a CO₂ incubator (37°C, 5%CO₂) to detach cells from the substrate.

### <<Step4: separation of substrate and cells, cell recovery>>

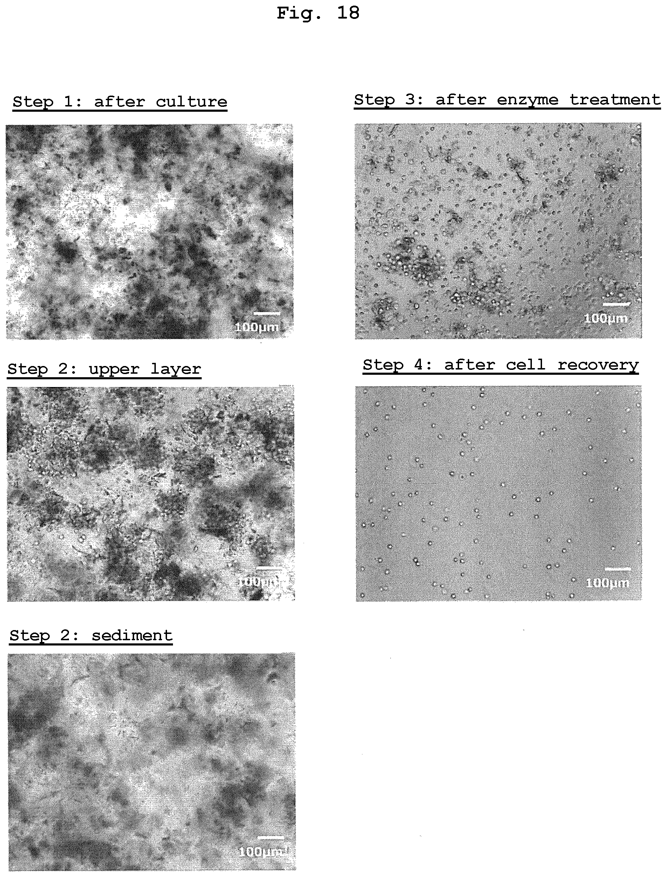

The medium (10 mL) was added to the obtained suspension and the mixture was filled in a 50 mL syringe (manufactured by Nipro), the syringe was connected to one end of a bone marrow fluid filtration filter (manufactured by KANEKA) which is one part of the commercially available product, and successively, the syringe was replaced with a 50 mL syringe filled with 25 mL of the medium, the liquid was sent from the lower part to the upper part direction, and the medium (total amount about 40 mL) coming out of the upper flow path was collected in a 50 mL conical tube. This was centrifuged (300×g, 3 min), and concentrated by removing the supernatant (35 mL), whereby the cells were recovered. The microscopic photographs of the suspensions obtained in each step of Steps 1 to 4 are shown in Fig. 18.

### [Industrial Applicability]

The present invention provides a separation device that more efficiently separates a separation target in a suspension culture of adherent cells using a fine scaffold material, and can obtain the passing target. Using the separation device, a method for preferably separating a separation target can be provided, the passing target can be preferably passed through, and the recovery target can be preferably recovered.

This application is based on a patent application No. 2018-170092 filed in Japan (filing date: September 11, 2018), the contents of which are incorporated in full herein.

### [Explanation of Symbols]

1 separation device
10 separation chamber
10a first chamber
10b second chamber
20 mesh structure for separation
F advancing direction of liquid passing through mesh-holes of mesh structure for separation
Fv direction component in vertically upward direction of advancing direction F

## Claims

1. A separation device for separating a solid separation target contained in a suspension which is obtained by suspension culture of adherent cells by using a fine scaffold material, the separation device comprising:
a separation chamber which comprises at least a first chamber and a second chamber, wherein the first chamber and the second chamber are divided by a mesh structure for separation, and an internal flow path is configured such that the liquid in the suspension medium that has entered the separation device moves from the first chamber through the mesh structure for separation to the second chamber and thereafter exits the separation device,
the mesh structure for separation has mesh-holes with a predetermined size to suppress passage of the separation target, and
the mesh structure for separation is configured in the separation chamber such that the advancing direction of the liquid in the aforementioned suspension has a vertically upward directional component when the liquid passes through the mesh-holes.

2. The separation device according to claim 1, wherein the suspension comprises a solid passing target to be passed through the mesh structure for separation together with the liquid, and
the mesh structure for separation has mesh-holes of a predetermined size that suppress passage of the separation target and allow passage of the passing target.

3. The separation device according to claim 2, wherein the liquid in the suspension is the liquid medium, the separation target is a scaffold material detached from the cells in the liquid medium by a reagent that separates the scaffold material and the adherent cell, and the passing target is the adherent cells detached from the scaffold material.

4. The separation device according to claim 3, wherein
the scaffold material is a nanofiber composed of a water-insoluble polysaccharide, and
the adherent cell is a cell selected from the group consisting of a dog kidney renal tubule epithelial cell (MDCK cell), a Chinese hamster ovary cell (CHO cell), and a mesenchymal stem cell (MSC).

5. The separation device according to claim 2, wherein
the liquid in the suspension is the liquid medium, the separation target is a scaffold material to which adherent cells suspension-cultured in the liquid medium are attached, and the passing target is a humoral factor released from the cells into the liquid medium by suspension culture.

6. The separation device according to claim 2, wherein
the liquid in the suspension is a liquid containing a cell lysate of suspension-cultured adherent cells, the separation target is a scaffold material left in the liquid by lysis of the cells, and the passing target is a substance derived from the adherent cell and released into the liquid by the lysis of the adherent cell.

7. The separation device according to any one of claims 1 to 6, wherein the mesh structure for separation is sheet-like, or has a shape of a bag.

8. The separation device according to any one of claims 1 to 7, wherein
the first chamber comprises
a first inlet port for flowing the suspension into the first chamber from the outside,
a second inlet port for flowing a washing solution or reagent into the first chamber from the outside, and
an outlet port for flowing the separation target from the first chamber to the outside.

9. The separation device according to any one of claims 1 to 8, wherein
the second chamber is located on the first chamber,
the first chamber has at least an inlet port for flowing the suspension from the outside, and the inlet port is provided on a side wall of the first chamber.

10. The separation device according to claim 9, wherein a central axis of the inlet port is at a height of not less than 40% of the whole height of the inside of the first chamber.

11. The separation device according to claim 9 or 10, further comprising one or more chambers stacked upwards as separation chambers on the second chamber, wherein
a chamber adjacent to the second chamber in the one or more chambers is divided from the second chamber by a further mesh structure for separation,
when the one or more chambers are two or more chambers, chambers adjacent to each other above and below are divided by a further different mesh structure for separation, and
mesh-holes of a mesh structure for separation located higher have a smaller opening area to suppress more the passage of the separation target.

12. The separation device according to claim 11, wherein the suspension comprises a solid passing target to be passed through the mesh structure for separation together with the liquid, and
a size of the mesh-holes of the mesh structure for separation located at the top is determined in advance to allow for the passage of the separation target.

13. A method for separating a solid separation target from a suspension by using the separation device according to any one of claims 1 to 12, wherein the suspension is obtained by suspension culturing adherent cells by using a fine scaffold material.

14. The method according to claim 13, wherein
the suspension comprises a solid passing target to be passed through the mesh structure for separation of the separation device together with the liquid,
the mesh structure for separation has mesh-holes of a predetermined size to prevent the separation target from passing through and allow the passing target to pass through, and
a step of recovering the passing target that passes through the mesh structure for separation is included.

15. The method according to claim 13 or 14, wherein a flow velocity of the liquid in the suspension passing through the mesh structure for separation of the separation device is 0.01 mm/sec - 25 mm/sec.
